(19) Europäisches Patentamt
European Patent Office
Office européen des brevets

(11) EP 4 703 365 A1

(12) EUROPEAN PATENT APPLICATION
published in accordance with Art. 153(4) EPC

(43) Date of publication:
**04.03.2026 Bulletin 2026/10**

(21) Application number: **24796011.5**

(22) Date of filing: **22.04.2024**

(51) International Patent Classification (IPC):
***C07D 513/14*** *(2006.01)* ***A61P 35/00*** *(2006.01)*
***A61K 31/551*** *(2006.01)*

(52) Cooperative Patent Classification (CPC):
**A61K 31/551; A61P 35/00; C07D 513/14**

(86) International application number:
**PCT/CN2024/089126**

(87) International publication number:
**WO 2024/222630 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.04.2023 CN 202310438327**

(71) Applicant: **Horizon Omics Biotech Limited
Beijing 100084 (CN)**

(72) Inventor: **XU, Hong
Beijing 100084 (CN)**

(74) Representative: **Müller-Boré & Partner
Patentanwälte PartG mbB
Friedenheimer Brücke 21
80639 München (DE)**

# (54) QUINOLONE DERIVATIVE AND USE THEREOF AGAINST TUMORS

(57) Provided are a quinolone analog as represented by formula (1), or a pharmaceutically acceptable salt, a stereoisomer, a racemate, a deuterated compound, or a solvent compound thereof, and a pharmaceutical composition thereof; and the use thereof in the preparation of a drug for treating tumors, autoimmune diseases, or diseases caused by bacteria, fungi or viruses.

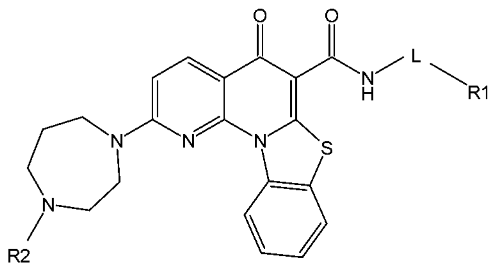

(I)

EP 4 703 365 A1

## Description

### REFERENCE TO RELATED APPLICATION

The present application claims the priority to and the benefits of Chinese Patent Application No. 202310438327.6, filed with the China National Intellectual Property Administration on April 23, 2023, the entire contents of which are incorporated herein by reference.

### TECHNICAL FIELD

The present application relates to a class of quinolone analogs and preparation methods and uses thereof, and belongs to the field of medicinal chemistry.

### BACKGROUND

Tetracycloquinolone compounds interact with quadruplex forming regions of nucleic acids and modulate the transcription of ribosomal RNAs. Specifically, tetracycloquinolone compounds can stabilize DNA G-quadruplexes (G4s) in cancer cells, thereby impeding DNA replication and translation, and even leading to DNA breaks and inducing synthetic lethality in cancer cells. Additionally, tetracycloquinolone compounds selectively inhibit rRNA synthesis mediated by Pol I within the nucleolus, leading to nucleolar fragmentation and subsequent killing cancer cells dependent on rRNA overexpression.

The tetracycloquinolone compound CX-5461 was previously considered as an RNA polymerase I inhibitor that suppresses rDNA translation. Some literatures have also suggested that CX-5461 has a topoisomerase-inhibiting effect.

Although CX-5461 has demonstrated preliminary efficacy in homologous recombination (HR)-deficient solid tumors, it requires high dosages in clinical trials. In the NCT02719977 clinical trial for treating solid tumors in humans, the recommended dosage of CX-5461 was 475 mg/$m^2$, and adverse effects such as phototoxicity, neutropenia, acral erythema, and nausea were observed.

The current clinical administration of CX-5461 involves multiple intravenous infusions, which must be performed in hospitals. This consumes substantial medical resources and is less convenient and cost-effective compared to oral administration. Furthermore, intravenous infusion introduces risks such as intravenous infusion-related infections, phlebitis, and thrombosis. Considering the underlying health conditions of cancer patients receiving CX-5461 treatment, as well as the outbreaks of epidemic infectious diseases (e.g., COVID-19, SARS) in recent years, it is highly necessary to develop relevant oral drugs that do not occupy valuable medical resources.

Currently, there is an urgent clinical need to enhance the tumor killing activity of compounds, improve their metabolic stability, reduce their toxic side effects, and increase their bioavailability, thereby developing the compounds into oral formulations that are more convenient for patients to use.

### SUMMARY OF INVENTION

In one aspect, the present application provides a quinolone analog represented by Formula (I), or a pharmaceutically acceptable salt, stereoisomer, racemate, deuterated compound, or solvate thereof:

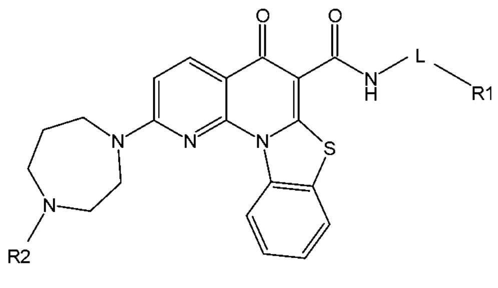

(I)

wherein, in the compound of Formula (I), R1 is selected from C1-C6 linear or branched alkyl, C5-C10 aryl, C5-C10 heteroaryl, C3-C8 cycloalkyl, or C3-C8 heterocycloalkyl, wherein the C1-C6 linear or branched alkyl, the C5-C10 aryl, the C5-C10 heteroaryl, the C3-C8 cycloalkyl, and the C3-C8 heterocycloalkyl are each independently optionally unsubstituted or substituted with a substituent, and the substituent is independently selected from oxo, C1-C4 alkyl, hydroxy(C1-C4 alkyl), halogen, cyano, hydroxyl, C1-C4 alkylamino, C1-C4 alkoxy, or halo(C1-C4 alkyl), and the heteroaryl is 5- to 10-membered heteroaryl containing one or more types of heteroatoms selected from N, O, or S with

a number of the heteroatoms from 1 to 3;

L is $-(CH_2)n-$, where n = 0-6;

R2 is selected from H, deuterium, C1-C4 alkyl, or C1-C4 alkyl substituted with one or more deuterium atoms.

In some embodiments, R1 is selected from C1-C6 linear or branched alkyl, C5-C10 aryl, or C5-C10 heteroaryl, wherein the C1-C6 linear or branched alkyl, the C5-C10 aryl, and the C5-C10 heteroaryl are unsubstituted or substituted with a substituent, and the substituent is independently selected from C1-C4 alkyl, hydroxy(C1-C4 alkyl), halogen, cyano, hydroxyl, C1-C4 alkylamino, C1-C4 alkoxy, or halo(C1-C4 alkyl), and the heteroaryl is 5- to 10-membered heteroaryl containing one or more types of heteroatoms selected from N, O, or S with a number of the heteroatoms of 1 to 3.

In some embodiments, R1 is selected from C1-C6 linear or branched alkyl, C5-C10 heteroaryl, C3-C8 cycloalkyl, or C3-C8 heterocycloalkyl, wherein the C1-C6 linear or branched alkyl, the C5-C10 heteroaryl, the C3-C8 cycloalkyl, and the C3-C8 heterocycloalkyl are each independently optionally unsubstituted or substituted with a substituent, and the substituent is independently selected from oxo, C1-C4 alkyl, hydroxy(C1-C4 alkyl), halogen, cyano, hydroxyl, C1-C4 alkylamino, C1-C4 alkoxy, or halo(C1-C4 alkyl), and the heteroaryl is 5- to 10-membered heteroaryl containing one or more types of heteroatoms selected from N, O or S with a number of the heteroatoms from 1 to 3.

In some embodiments, R1 is selected from methyl,

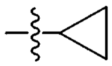, 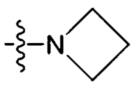, 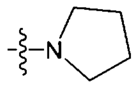,

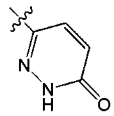, or 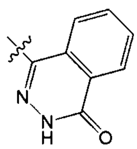.

In some embodiments, L is $-(CH_2)_n-$, where n = 0-3. In some embodiments, L is $-(CH_2)_n-$, where n = 0-2. In some embodiments, L is $-CH_2-$ or $-CH_2CH_2-$. In some embodiments, L is $-(CH_2)_n-$, where n = 0 (i.e., L is absent, and NH is directly connected to R1 through a bond).

In some embodiments, R2 is selected from H, deuterium, or C1-C4 alkyl substituted with one or more deuterium atoms. In some embodiments, R2 is selected from C1-C4 alkyl. In some embodiments, R2 is methyl.

The quinolone compounds of the present application comprise the following Compound C to Compound F, Compound G-1 and Compound G-2),

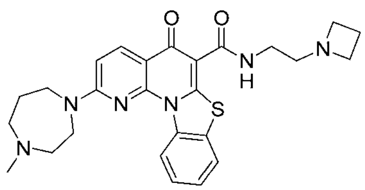 C, 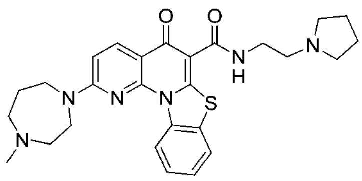 D, 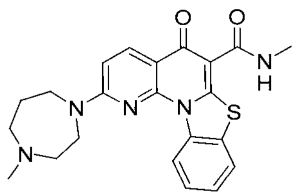 E,

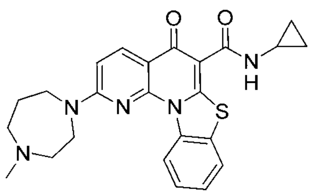 F, 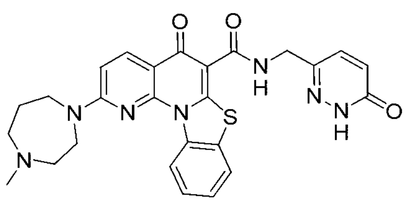 G-1, 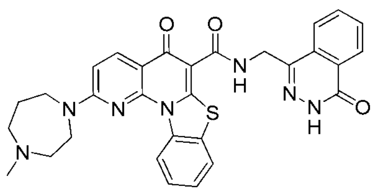 G-2.

Further, the compounds also comprise the pharmaceutically acceptable salts, stereoisomers, racemates, deuterated compounds, or solvates thereof.

Further, the synthesis route of Compound **C** is as follows:

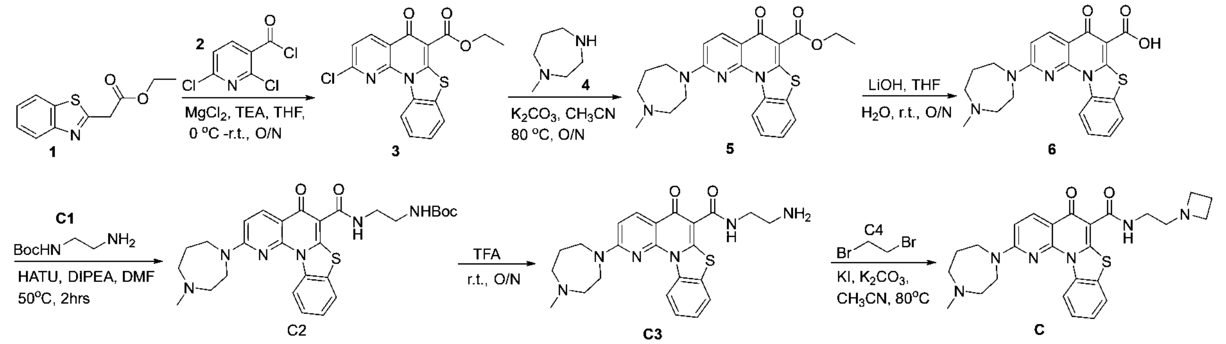


Compound **1** reacts with 2,6-dichloropyridine-3-carbonyl chloride to generate Compound **3;** Compound **3** then reacts with N-methylhomopiperazine under an alkaline condition to generate Compound **5;** Compound **5** is then hydrolyzed with lithium hydroxide to obtain Compound **6;** Compound **6** undergoes a condensation reaction with Compound **C1** under the action of HATU to form Compound **C2,** where "O/N" denotes overnight preparation; Compound C2 is dissolved in a trifluoroacetic acid/dichloromethane solution to remove the protecting group, yielding Compound **C3;** and Compound **C3** reacts with 1,3-dibromopropane (Compound **C4**) to obtain the target Compound **C.**

The synthesis route of Compound **D** is as follows:

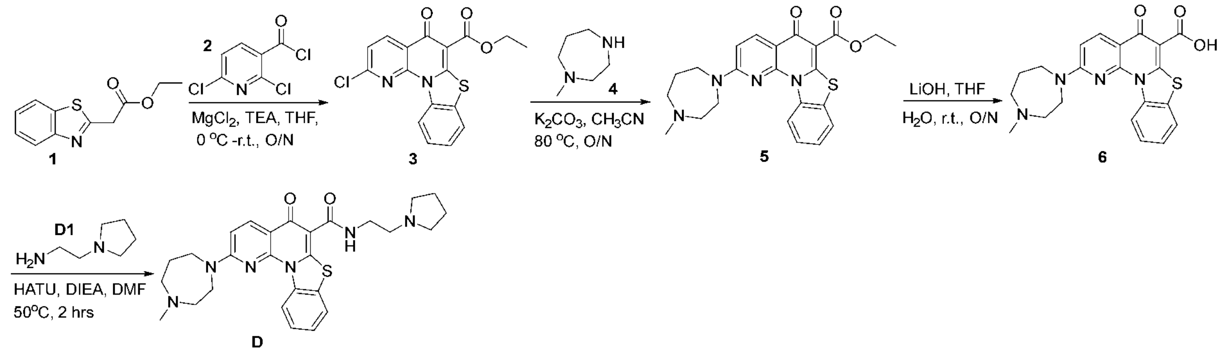


Compound **6** is prepared according to the aforementioned method; Compound 6 undergoes a condensation reaction with 1-pyrrolidineethanamine (Compound **D1**) under the action of 2-(7-azabenzotriazol-1-yl)-1,1,3,3-tetramethyluronium hexafluorophosphate (HATU) to generate Compound **D.**

The synthesis route of Compound **E** is as follows:

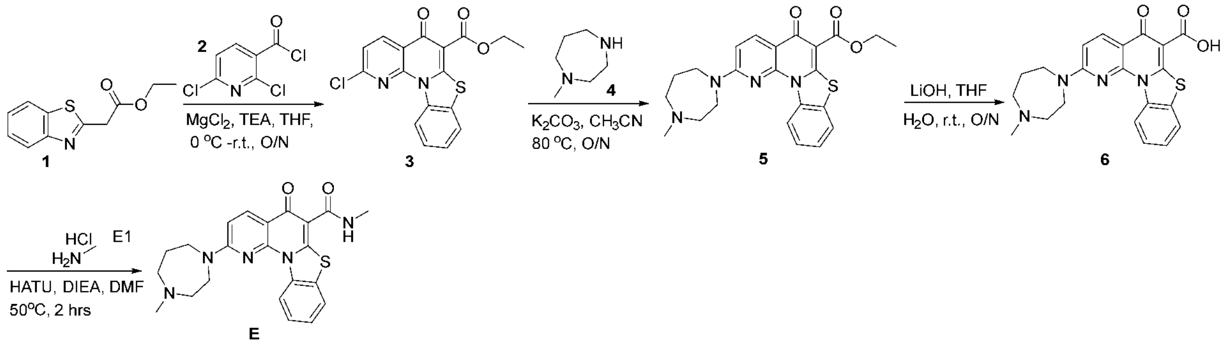


Compound **6** is prepared according to the aforementioned method; and Compound **6** undergoes a condensation reaction with methylamine hydrochloride (Compound **E1**) under the action of 2-(7-azabenzotriazol-1-yl)-1,1,3,3-tetramethyluronium hexafluorophosphate (HATU) to generate Compound **E.**

The synthesis route of Compound F is as follows:

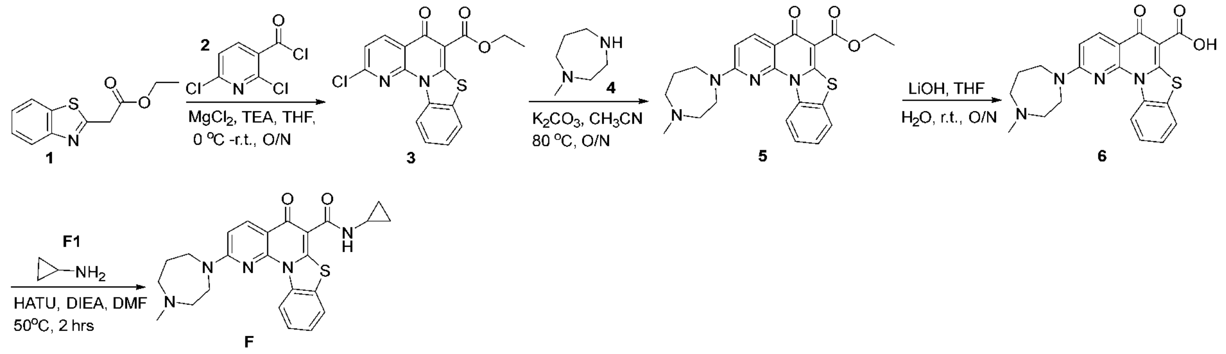


Compound **6** is prepared according to the aforementioned method; and Compound **6** undergoes a condensation reaction with cyclopropylamine (Compound **F1**) under the action of 2-(7-azabenzotriazol-1-yl)-1,1,3,3-tetramethyluronium hexafluorophosphate (HATU) to generate Compound **F.**

The synthesis route of Compound G-1 is as follows:

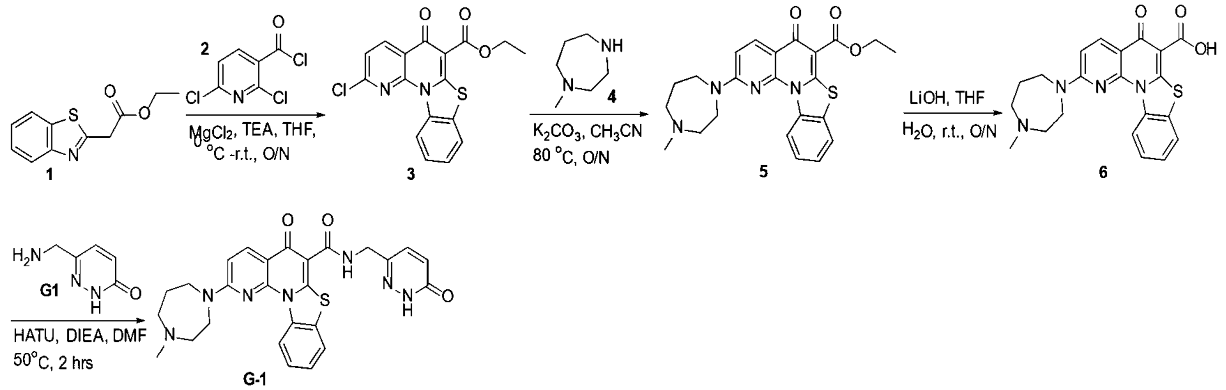


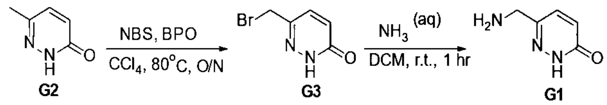


Compound 6 is prepared according to the aforementioned method; and Compound 6 undergoes a condensation reaction with Compound **G1** under the action of 2-(7-azabenzotriazol-1-yl)-1,1,3,3-tetramethyluronium hexafluorophosphate (HATU) to generate Compound **G-1.**

The synthesis route of Compound **G-2** is as follows:

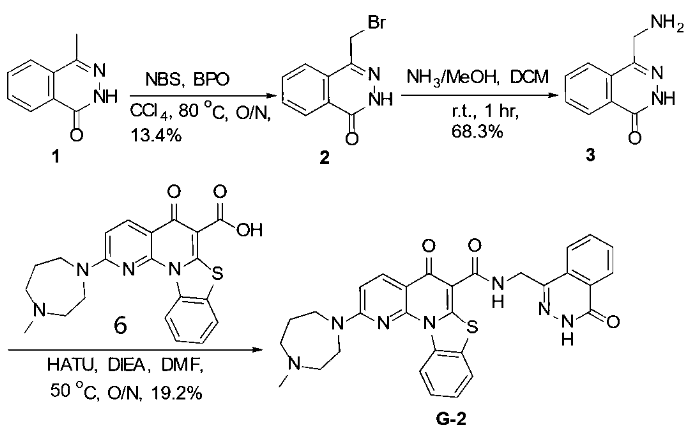


The "pharmaceutically acceptable salt" of the compound represented by Formula (I) in the present application usually exists in the form of a free acid. The acid addition salt of the free amino compound of the present application can be prepared by methods known in the art. Suitable acids include, but are not limited to, phosphoric acid, hydrochloric acid,

hydrobromic acid, hydrofluoric acid, hydroiodic acid, sulfuric acid, nitric acid, maleic acid, fumaric acid, benzoic acid, ascorbic acid, succinic acid, methanesulfonic acid, formic acid, acetic acid, trifluoroacetic acid, oxalic acid, propionic acid, tartaric acid, salicylic acid, citric acid, gluconic acid, lactic acid, mandelic acid, phenylacetic acid, aspartic acid, stearic acid, palmitic acid, glycolic acid, glutamic acid, p-toluenesulfonic acid, and benzenesulfonic acid. In addition, groups containing a basic nitrogen atom can be quaternized with the following reagents: lower alkyl halides (e.g., chlorides, bromides, and iodides of methyl, ethyl, and butyl); dialkyl sulfates (e.g., dimethyl sulfate, diethyl sulfate, dibutyl sulfate, and dipentyl sulfate); long-chain halides (e.g., chlorides, bromides, and iodides of decyl, dodecyl, tetradecyl, and octadecyl); arylalkyl halides (e.g., benzyl bromide and phenylethyl bromide), and the like. The resulting products are water-soluble, oil-soluble, or dispersible. Therefore, "pharmaceutically acceptable salt" shall include all acceptable salt forms.

The "stereoisomer" and "racemate" of the compound represented by Formula (I) of the present application generally mean that the compound of Formula (I) may have a chiral center and exist in the form of a racemate, a racemic mixture, and individual enantiomer or diastereomer. All isomeric forms, including their mixtures, are included within the scope of the present application.

In addition, some compounds represented by Formula (I) may also form solvates with water or other organic solvents, such as hydrates and tert-butanol solvates. Such solvates are also similarly included within the scope of the present application.

Those skilled in the art will understand that any compound may contain non-natural proportions of atomic isotopes at one or more atoms constituting the compound. In the context of the present application, said "deuterated" refers to the presence of deuterium atoms at relevant sites of the compound in proportions exceeding the natural proportion (i.e., exceeding the natural abundance of deuterium). Therefore, any compound represented by Formula (I) that contains deuterium atoms at relevant positions in proportions higher than the natural abundance of deuterium falls within the protection scope of the present application. For example, it should be understood that compounds represented by Formula (I) with corresponding deuteration ratios or deuterium contents, obtained by introducing deuterium atoms using commercially available deuteration reagents through chemical synthesis methods that are the same as or similar to those shown in the examples of the present application, are all within the protection scope of the present application. The chemical synthesis methods and deuteration reagents herein are not limited to those exemplified in the examples, but shall be understood to encompass all synthesis methods or routes available in the field for obtaining the compounds of the present application, and all deuteration reagents that can be used in conjunction with the aforementioned synthesis methods or routes to introduce deuterium atoms into the target molecules.

In another aspect, the present application provides a pharmaceutical composition comprising the compound or the pharmaceutically acceptable salt, stereoisomer, racemate, deuterated compound, or solvate thereof described in the present application, as an active ingredient.

In another aspect, the present application provides a pharmaceutical composition comprising a therapeutically effective amount of the compound or the pharmaceutically acceptable salt, stereoisomer, racemate, deuterated compound, or solvate thereof described in the present application, and one or more pharmaceutically acceptable carriers or excipients.

In another aspect, the present application provides use of the compound or the pharmaceutically acceptable salt, stereoisomer, racemate, deuterated compound, or solvate thereof described in the present application or the pharmaceutical composition described in the present application in the preparation of a medicament for the treatment of tumors, autoimmune diseases, or diseases caused by bacteria, fungi, or viruses.

In another aspect, the present application provides the compound or the pharmaceutically acceptable salt, stereoisomer, racemate, deuterated compound, or solvate thereof described in the present application or the pharmaceutical composition described in the present application for use in the treatment of tumors, autoimmune diseases, or diseases caused by bacteria, fungi, or viruses.

In a further aspect, the present application provides a method for treating tumors, autoimmune diseases, or diseases caused by bacteria, fungi, or viruses, the method comprising administering to a patient a therapeutically effective amount of the compound or the pharmaceutically acceptable salt, stereoisomer, racemate, deuterated compound, or solvate thereof described in the present application or the pharmaceutical composition described in the present application.

In a further aspect, the present application provides use of the compound or the pharmaceutically acceptable salt, stereoisomer, racemate, deuterated compound, or solvate thereof described in the present application or the pharmaceutical composition described in the present application in the treatment of tumors, autoimmune diseases, or diseases caused by bacteria, fungi, or viruses.

## Method of Use

The present application provides use of a quinolone analog represented by Formula (I) or a pharmaceutically acceptable salt, stereoisomer, racemate, deuterated compound, or solvate thereof in the preparation of a medicament.

The present application provides uses of the aforementioned compound or the pharmaceutically acceptable salt thereof in the preparation of a medicament for treating cancers with BRCA-1 or BRCA-2 mutations (homozygous or heterozygous mutations), homologous recombination repair-deficient cancers, non-homologous end joining (NHEJ) repair-deficient cancers, or other DNA damage repair-deficient cancers; and in the preparation of a medicament for treating cancers with overexpression of c-Myc, N-Myc, or L-Myc, cancers with overexpression of other oncogenes (HIF, VEGF, ABL, TGF, PDGF, MYB, SPARC, HER2, C-KIT1, C-KIT2, VAV, RET, N-RAS, H-RAS, K-RAS, EGF, SRC, BCL-1, BCL-2, DHFR, HMGA, etc.), and cancers with an abnormal chromosome number. Use alone and in combination therapy is included. The combination therapy may be performed by simultaneous or sequential administration.

The present application provides the use of the aforementioned compound or the pharmaceutically acceptable salt thereof in the preparation of a medicament for treating diseases that are ameliorated or rendered resistant by the use of a PARP inhibitor, or the use of the aforementioned compound or the pharmaceutically acceptable salt thereof in combination with a PARP inhibitor drug. The PARP inhibitor drug used in combination is selected from Olaparib, Niraparib, Rucaparib, Talazoparib, Fluzoparib, Pamiparib, or other PARP inhibitors.

The present application provides use of the aforementioned compound or the pharmaceutically acceptable salt thereof in combination with topoisomerase I and topoisomerase II inhibitors, CHK1 inhibitors, CHK2 inhibitors, ATM inhibitors, ATR inhibitors, DNA-pK inhibitors, WEE1 inhibitors, various DNA polymerases, RNA polymerase inhibitors, targeted drugs for the DNA damage repair (DDR) network, mTORC1/2 inhibitors, histone deacetylase inhibitors, proteasome inhibitors, RNA transcription inhibitors, mRNA translation inhibitors, PIM kinase and other kinase inhibitors, or p53 activators.

The present application also provides use of the aforementioned compound or the pharmaceutically acceptable salt thereof in the preparation of adjuvant drugs for tumor treatment.

Further, the present application also provides use of the aforementioned compound or the pharmaceutically acceptable salt thereof in combination with chemotherapy, radiotherapy, targeted therapy, immunotherapy, immune checkpoint inhibitors (such as PD-1, PD-L1, and CTLA-4 inhibitors), endocrine therapy, metabolic therapy, oncolytic virus therapy, or other therapies for treating cancer.

Further, the cancer is breast cancer, ovarian cancer, endometrial cancer, cervical cancer, oral cancer, pancreatic cancer, prostate cancer, brain cancer, lung cancer, liver cancer/hepatocellular carcinoma (HCC), leukemia, lymphoma, myeloma, multiple myeloma, skin cancer, peritoneal cancer, colorectal cancer, glioblastoma, melanoma, osteosarcoma, cervical cancer, Ewing's sarcoma, lymph node cancer, gastrointestinal malignancy, head and neck cancer, kidney cancer, cardiac cancer, or other cancers.

The present application provides use of the aforementioned compound or the pharmaceutically acceptable salt thereof in the preparation of a medicament for treating autoimmune deficiency diseases such as multiple sclerosis, either alone or in combination therapy. The combination therapy can be performed by simultaneous or sequential administration.

The present application provides use of the aforementioned compound or the pharmaceutically acceptable salt thereof in the preparation of a medicament for treating diseases caused by bacteria, fungi, or viruses. Viral infections include, but are not limited to, hepatitis B virus, hepatitis C virus, rhinovirus, varicella-zoster virus, herpes simplex virus, cytomegalovirus, poxvirus, encephalitis virus, hantavirus, arbovirus, human papillomavirus (HPV), Sironi virus, human immunodeficiency virus, influenza virus, Epstein-Barr (EB) virus, respiratory syncytial virus, coronaviruses (SARS-CoV, SARS-CoV-2, MERS-CoV), and the like. Use alone and in combination therapy are included. The combination therapy can be performed by simultaneous or sequential administration.

Formulations

The formulations of the present application are prepared by using the compound represented by Formula (I) or the pharmaceutically acceptable salt, stereoisomer, racemate, deuterated compound, or solvate thereof as the active ingredient, together with pharmaceutically acceptable excipients or auxiliary ingredients. The pharmaceutically acceptable auxiliary ingredients have certain physiological activities, but their addition does not alter the dominant role of the pharmaceutical composition in disease treatment and only exerts auxiliary effects. These auxiliary effects are merely the utilization of the known activities of the ingredients and are conventional adjuvant therapeutic methods in the medical field. The combined use of the auxiliary ingredients with the pharmaceutical composition of the present application shall still fall within the protection scope of the present application.

The term "pharmaceutically acceptable carrier" refers to one or more compatible solid or liquid fillers or gel substances that are suitable for human use, and must have sufficient purity and sufficiently low toxicity. Examples of the pharmaceutically acceptable carrier include, but are not limited to, cellulose and its derivatives (e.g., sodium carboxymethyl cellulose, sodium ethyl cellulose, cellulose acetate, etc.), gelatin, talc, solid lubricants (e.g., stearic acid, magnesium stearate), calcium sulfate, vegetable oils (e.g., soybean oil, sesame oil, peanut oil, olive oil, etc.), polyols (e.g., propylene glycol, glycerol, mannitol, sorbitol, etc.), emulsifiers, wetting agents, colorants, flavoring agents, stabilizers, antioxidants, preservatives, pyrogen-free water, and any other types of non-toxic solid, semi-solid, and liquid

fillers, diluents, encapsulating materials, and formulation adjuvants.

Further, the present application provides a pharmaceutical composition comprising a therapeutically effective amount of the quinolone compound or the pharmaceutically acceptable salt, stereoisomer, racemate, deuterated compound, or solvate thereof as an active ingredient, and a pharmaceutically acceptable carrier.

Pharmaceutical Composition

The pharmaceutical composition of the present application may be in a solid or liquid form. On one hand, the carrier is microparticles, so that the composition is, for example, in the form of tablets or powders. The carrier may be a liquid, and the composition is, for example, an oral syrup, an injectable liquid, or an aerosol suitable for administration by inhalation. When intended for oral administration, the pharmaceutical composition is preferably in a solid or liquid form. The semi-solid, semi-liquid, suspension, and gel forms are included herein as the solid or liquid forms. For oral solid compositions, the pharmaceutical composition may be formulated into powders, granules, compressed tablets, pills, capsules, chewable tablets, powder tablets, and the like. Such solid compositions usually contain one or more inert diluents or edible carriers. In addition, one or more of the following substances may also be present: binders, e.g., carboxymethyl cellulose, ethyl cellulose, microcrystalline cellulose, xanthan gum/tragacanth, or gelatin; excipients, e.g., starch, lactose, or dextrin; disintegrants, e.g., alginic acid, sodium alginate, corn starch, etc.; lubricants, e.g., magnesium stearate or hydrogenated vegetable oil; glidants, e.g., colloidal silicon dioxide; sweeteners, e.g., sucrose or saccharin; flavoring agents, e.g., peppermint, methyl salicylate, or sweet orange flavor; and colorants.

The pharmaceutical composition may be administered parenterally, intracisternally, vaginally, intraperitoneally, topically (e.g., powders, ointments, drops, and transdermal patches), rectally, or buccally. As used herein, the term "parenteral" refers to an administration mode including intravenous, intramuscular, intraperitoneal, intra sternal, subcutaneous, and intra-articular injections and infusions. The pharmaceutical composition for parenteral injection includes pharmaceutically acceptable sterile aqueous or non-aqueous solutions, dispersions, suspensions, and emulsions, as well as sterile powders for reconstitution into sterile injection solutions or dispersions immediately before use. Suitable aqueous and non-aqueous carriers, diluents, solvents, or excipients include water, ethanol, polyols (e.g., glycerol, propylene glycol, polyethylene glycol, etc.), carboxymethyl cellulose, and suitable mixtures thereof, vegetable oils (e.g., olive oil), and injectable organic esters (e.g., ethyl oleate). In the case of dispersions, the required particle size may be maintained by using coating materials such as lecithin, and the suitable fluidity may be maintained by using surfactants.

When the pharmaceutical composition is in a capsule form (e.g., gelatin capsules), in addition to the above-mentioned types of substances, it may also contain a liquid carrier such as polyethylene glycol or oil. The pharmaceutical composition may be in a liquid form, such as tinctures, syrups, solutions, emulsions, or suspensions. When administered orally, the composition preferably contains, in addition to the compound of the present application, one or more of sweeteners, preservatives, dyes/colorants, and flavor enhancers. For compositions intended for injection, one or more of surfactants, preservatives, wetting agents, dispersants, suspending agents, buffers, stabilizers, and isotonic agents may be included.

The pharmaceutical composition of the present application may include various substances that modify the physical form of solid or liquid dosage units.

The pharmaceutical composition of the present application in a solid or liquid form may include an agent that binds to the compound of the present application and thereby aids in the delivery of the compound. Suitable agents with this capability include monoclonal or polyclonal antibodies, proteins, or liposomes.

The pharmaceutical composition of the present application may be prepared by using methods well-known in the pharmaceutical field. For example, the pharmaceutical composition intended for administration by injection may be prepared by combining the compound of the present application with sterile distilled water to form a solution. A surfactant may be added to form a homogeneous solution or suspension. The surfactant is a compound that interacts non-covalently with the compound of the present application, thereby promoting the dissolution or uniform suspension of the compound of the present application in an aqueous delivery system.

The compound represented by Formula (I) or the pharmaceutically acceptable salt, stereoisomer, racemate, deuterated compound, or solvate thereof of the present application is administered in a therapeutically effective amount, which will vary depending on a variety of factors, including the activity of the specific compound used; the metabolic stability and duration of action of the compound; the age, weight, general health status, gender, diet, mode and timing of administration, excretion rate, drug combination, severity of the specific condition or disorder of the patient; and the individual receiving the therapy.

The compound represented by Formula (I) or the pharmaceutically acceptable salt, stereoisomer, racemate, deuterated compound, or solvate thereof of the present application may also be administered simultaneously with, before, or after the administration of one or more other therapeutic agents. This combination therapy includes the administration of a single pharmaceutical dosage formulation containing the compound of the present application and one or more

additional active agents, as well as the administration of the compound represented by Formula (I) of the present application with separate pharmaceutical dosage formulations of each active agent.

A medical composition consists of the compound represented by Formula (I) or the pharmaceutically acceptable salt, stereoisomer, racemate, deuterated compound, or solvate thereof of the present application, combined with an immunomodulator, an antibiotic (e.g., penicillins, aminoglycosides, quinolones, macrolides, etc.), and an antiviral drug (e.g., neuraminidase inhibitors, cap-dependent endonuclease inhibitors, RNA-dependent RNA polymerase inhibitors, M2 protein inhibitors, etc.).

Definitions and Explanations

The definitions of groups and terms described in the present application, including those defined as examples, exemplary definitions, preferred definitions, definitions of specific compounds in the examples, etc., may be arbitrarily combined with each other.

A specific term shall not be considered uncertain or unclear if it is not specifically defined, but shall be understood in accordance with its ordinary meaning in the art. When a trade name appears herein, it is intended to refer to the corresponding commodity or its active ingredient.

Unless otherwise defined, the following terms used in the description and claims have the meanings set forth below.

The term "stereoisomer" refers to isomers resulting from different spatial arrangements of atoms in a molecule, including cis-trans isomers, enantiomers, and diastereomers.

The term "pharmaceutical composition" refers to a mixture of one or more of the compounds of the present application with additional chemical components, such as a pharmaceutically acceptable carrier. The pharmaceutical composition is intended to facilitate the process of administering the compound to animals.

The term "pharmaceutical carrier" refers to an inactive ingredient in the pharmaceutical composition that does not cause significant irritation to the organism and does not interfere with the biological activity and properties of the administered compound, such as, but not limited to, calcium carbonate, various sugars (e.g., lactose, mannitol, etc.), starch, cyclodextrins, magnesium stearate, cellulose, gelatin, water, polyethylene glycol, propylene glycol, ethylene glycol, castor oil or hydrogenated castor oil or polyethoxylated hydrogenated castor oil, sesame oil, corn oil, peanut oil, and the like.

The term "substituted" means that any one or more hydrogen atoms on a specific atom are replaced by substituents, provided that the valence state of the specific atom is normal and the substituted compound is stable. When the substituent is oxo (i.e., =O), it means that two hydrogen atoms are replaced, and oxo does not occur on aromatic groups. Unless otherwise specified, the term "substituted" refers to any level of substitution, such as monosubstituted, di-substituted, tri-substituted, tetra-substituted, or penta-substituted, as long as such substitution is permitted. The substituents are selected independently, and substitution may occur at any chemically accessible position. It should be understood that substitution on a given atom is limited by valence. It should also be understood that the substitution on a given atom results in a chemically stable molecule.

The term "optional" or "optionally" means that the subsequent described event or situation may or may not occur, and the description includes both the occurrence and non-occurrence of the event or situation. For example, ethyl being "optionally" substituted with halogen, means that ethyl may be unsubstituted ($CH_2CH_3$), monosubstituted (e.g., $CH_2CH_2F$, $CH_2CH_2Cl$), poly-substituted (e.g., $CHFCH_2F$, $CH_2CHF_2$, $CHFCH_2Cl$, $CH_2CHCl_2$), or fully substituted (e.g., $CF_2CF_3$, $CF_2CCl_3$, $CCl_2CCl_3$). Those skilled in the art will understand that for any group containing one or more substituents, no substitution or substitution pattern that is sterically impossible and/or cannot be synthesized will be introduced.

When any variable (e.g., B) appears more than once in the composition or structure of a compound, its definition in each case is independent. For example, if a group is substituted with 2 $R^b$, each $R^b$ has independent options.

When a bond of a substituent is cross-connected to two atoms on a ring, the substituent may be bonded to any atom on the ring.

The term "pharmaceutically acceptable" refers to those compounds, materials, compositions, and/or dosage forms that, within the scope of sound medical judgment, are suitable for use in contact with human and animal tissues without excessive toxicity, irritation, allergic reactions, or other problems or complications, and are commensurate with a reasonable benefit-risk ratio.

The term "alkyl", used alone or in combination with other terms, refers to a saturated hydrocarbon group that may be linear or branched. The term "Cn-Cm alkyl" refers to an alkyl group having n to m carbon atoms. In some embodiments, the alkyl group contains 1 to 6 carbon atoms, 1 to 4 carbon atoms, 1 to 3 carbon atoms, or 1 to 2 carbon atoms. Examples of an alkyl include, but are not limited to, methyl, ethyl, n-propyl, isopropyl, n-butyl, tert-butyl, isobutyl, sec-butyl, 2-methyl-1-butyl, n-pentyl, 3-pentyl, n-hexyl, 1,2,2-trimethylpropyl, and the like.

The term "alkylene", used alone or in combination with other terms, refers to a divalent alkyl linking group. The term "Cn-Cm alkylene" refers to an alkylene group having n to m carbon atoms. In some embodiments, the alkylene group

contains 1 to 6 carbon atoms, 1 to 4 carbon atoms, 1 to 3 carbon atoms, or 1 to 2 carbon atoms. Examples of the alkylene group include, but are not limited to, methylene, ethylene, propylene, ethane-1,2-diyl, ethane-1,1-diyl, propane-1,3-diyl, propane-1,2-diyl, propane-1,1-diyl, butane-1,4-diyl, butane-1,3-diyl, butane-1,2-diyl, 2-methyl-propane-1,3-diyl, and the like.

The term "oxo" refers to=O.

The term "cyano" or "nitrile" refers to a group of the formula -C≡N, which can also be written as -CN.

The term "halo" or "halogen", used alone or in combination with other terms, refers to fluorine, chlorine, bromine, and iodine. In some embodiments, "halo" refers to a halogen atom selected from F or Cl. In some embodiments, the halo group is F.

As used herein, the term "haloalkyl" refers to an alkyl group in which one or more hydrogen atoms are replaced by halogen atoms. The term "Cn-Cm haloalkyl" refers to a Cn-Cm alkyl group having n to m carbon atoms and containing at least 1 to at most {2(n to m)+1} halogen atoms, where the halogen atoms may be the same or different. In some embodiments, the halogen atoms are fluorine atoms. In some embodiments, the haloalkyl group has 1 to 6, or 1 to 4 carbon atoms. Examples of the haloalkyl group include $CF_3$, $C_2F_5$, $CH_2CF_3$, $CHF_2$, $CH_2F$, $CCl_3$, $CHCl_2$, $C_2Cl_5$, etc. In some embodiments, the haloalkyl group is trifluoromethyl ($CF_3$).

The term "aryl", used alone or in combination with other terms, refers to an aromatic hydrocarbon group, which may be monocyclic or polycyclic (e.g., having 2 fused rings). The term "Cn-Cm aryl" refers to an aryl group having n to m ring carbon atoms. The aryl group includes, for example, phenyl, naphthyl, and the like. In some embodiments, the aryl group has 5 to 10 carbon atoms. In some embodiments, the aryl group has 6 carbon atoms. In some embodiments, the aryl group has 10 carbon atoms. In some embodiments, the aryl group is phenyl.

The term "heteroaryl", used alone or in combination with other terms, refers to a monocyclic or polycyclic aromatic heterocyclic ring containing at least one heteroatom ring member selected from the group consisting of sulfur, oxygen, and nitrogen. In some embodiments, the heteroaryl ring has 1, 2, 3, or 4 heterocyclic ring members independently selected from nitrogen, sulfur, and oxygen. In some embodiments, the heteroaryl has 5 to 10 ring atoms (including carbon atoms) and 1, 2, 3, or 4 heteroatom ring members independently selected from the group consisting of nitrogen, sulfur, and oxygen. In some embodiments, the heteroaryl has 5 to 6 ring atoms and 1 or 2 heteroatom ring members independently selected from the group consisting of nitrogen, sulfur, and oxygen. In some embodiments, the heteroaryl is a 5-membered or 6-membered heteroaryl ring. In other embodiments, the heteroaryl is an 8-membered, 9-membered, or 10-membered fused bicyclic heteroaryl ring. Examples of the heteroaryl include, but are not limited to, thienyl, furyl, pyrrolyl, oxazolyl, thiazolyl, imidazolyl, pyrazolyl, isoxazolyl, isothiazolyl, oxadiazolyl, triazolyl, tetrazolyl, thiadiazolyl, and their benzo derivatives (e.g., benzofuryl, benzothienyl, benzothiazolyl, benzoxazolyl, benzisoxazolyl, benzimidazolyl, benzotriazolyl, indazolyl, indolyl, isoindolyl, etc.); or pyridyl, pyridazinyl, pyrimidinyl, pyrazinyl, triazinyl, and their benzo derivatives (e.g., benzopyridazinyl, quinolyl, quinazolinyl, isoquinolyl, etc.); or azocinyl, indolizinyl, purinyl, and their benzo derivatives; or cinnolinyl, phthalazinyl, quinazolinyl, quinoxalinyl, naphthyridinyl, pteridinyl, carbazolyl, acridinyl, phenazinyl, phenothiazinyl, phenoxazinyl, etc. The term "5-6 membered heteroaryl" refers to an aromatic ring system having 5 or 6 ring atoms, which contains 1 to 3, preferably 1 or 2, heteroatoms independently selected from the group consisting of N, O, and S.

A 5-membered heteroaryl ring refers to a heteroaryl group with five ring atoms, where one or more (e.g., 1, 2, or 3) of the ring atoms are independently selected from the group consisting of N, O, and S.

A 6-membered heteroaryl ring refers to a heteroaryl group with six ring atoms, where one or more (e.g., 1, 2, or 3) of the ring atoms are independently selected from the group consisting of N, O, and S.

The term "cycloalkyl" refers to a fully saturated carbocyclic ring that may exist as a monocyclic ring, bridged ring, or spiro ring. Unless otherwise indicated, the carbocyclic ring is typically a 3- to 8-membered ring (e.g., a 3-to 6-membered ring). Non-limiting examples of cycloalkyl include, but are not limited to, cyclopropyl, cyclobutyl, cyclopentyl, cyclohexyl, and the like.

The term "heterocycloalkyl" refers to a fully saturated cyclic group that may exist as a monocyclic ring, bridged ring, or spiro ring. Unless otherwise indicated, the heterocyclic ring is generally a 3- to 8-membered ring, 3- to 7-membered ring, 3- to 6-membered ring, 5- to 6-membered ring, 4- to 5-membered ring, or 3- to 5-membered ring containing 1 to 3 heteroatoms independently selected from boron, sulfur, oxygen, and/or nitrogen (preferably 1, 2, or 3 heteroatoms, with the heteroatoms preferably selected from the group consisting of nitrogen, oxygen, and sulfur). Examples of 3-membered heterocycloalkyl include, but are not limited to, epoxyethanyl, cyclothioethanyl, and cyclonitroethanyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-thioxanyl, 1,4-dioxanyl, thiomorpholinyl, 1,3-dithianyl, and 1,4-dithianyl; examples of 7-membered heterocycloalkyl include, but are not limited to, azacycloheptyl, oxacycloheptyl, and thiacycloheptyl. Preferred are monocyclic heterocycloalkyl groups having 4, 5, or 6 ring atoms.

As used herein, the expressions "ambient temperature" and "room temperature" are understood in the art and

generally refer to a temperature, such as a reaction temperature, that is approximately the temperature of the room where the reaction is conducted, for example, a temperature ranging from about 20°C to about 30°C.

The present invention also includes pharmaceutically acceptable salts of the compounds described herein. The term "pharmaceutically acceptable salt" refers to a derivative of the disclosed compound, where the parent compound is modified by converting the existing acidic or basic moiety into its salt form. The pharmaceutically acceptable salts of the present invention include, for example, non-toxic salts of the parent compound formed with non-toxic inorganic or organic acids. The pharmaceutically acceptable salts of the present invention may be synthesized from the parent compound containing a basic or acidic moiety through conventional chemical methods.

As used herein, the term "heteroatom" shall be understood to mean an atom other than carbon, such as O, N, or S.

## DESCRIPTION OF EMBODIMENTS

The present application is further described below in conjunction with the examples, but these examples are not intended to limit the scope of the present application.

For the experimental methods in the examples of the present application where specific conditions are not indicated, conventional conditions are generally followed; for reagents where specific sources are not indicated, they are conventional commercially available reagents.

The present application is further illustrated below in conjunction with examples:

Example 1: Preparation of Compound C

Step 1: Preparation of 2,6-dichloropyridine-3-carbonyl chloride

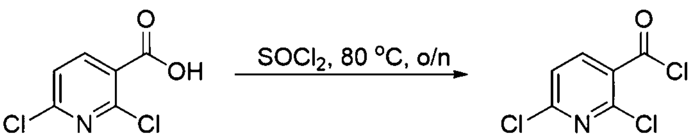


The compound 2,6-dichloropyridine-3-carboxylic acid (5 g, 26.0 mmol, 1.0 eq.) was added to thionyl chloride (40 mL) under ice-bath cooling, and then the system was heated to 80°C and reacted overnight (o/n, same applies below). Thionyl chloride was removed under vacuum to obtain 2,6-dichloropyridine-3-carbonyl chloride (5.4 g, a yellow oil substance, 98% yield). LC-MS (ESI): m/z 206.00 $[M+1]^+$ (the acyl chloride was quenched with methanol, and the MS shows the methyl ester of the indicated product), $^1$HNMR (400 MHz, $CDCl_3$) δ: 8.40 - 8.30 (m, 1H), 7.50 - 7.40 (m, 1H).

Step 2: Preparation of Compound **3**

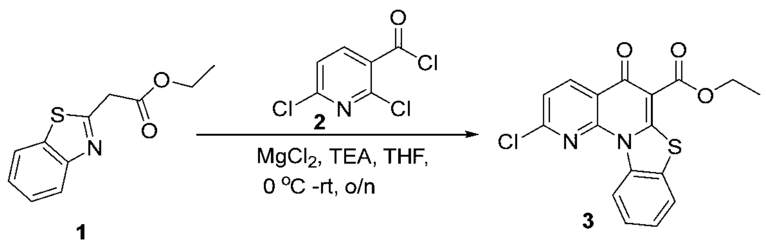


1 3

Compound **1** (1.0 g, 4.5 mmol, 1.0 eq.) was dissolved in tetrahydrofuran (10 mL), and then anhydrous magnesium chloride (430 mg, 4.5 mmol, 1.0 eq.) was added to the system. Under nitrogen protection, the reaction mixture was stirred at 0°C for half an hour. Then 2,6-dichloropyridine-3-carbonyl chloride (Compound **2,** 2.8 g, 13.5 mmol, 3.0 eq.) and triethylamine (2.3 g, 22.6 mmol, 5.0 eq.) were slowly added to the system respectively. The mixture reacted overnight at room temperature. The reaction mixture was filtered directly, and the filter cake was washed with water and methanol, and then dried to obtain Compound **3** (1.2 g, a light yellow solid, 68% yield). LC-MS (ESI): m/z 359.2 $[M+1]^+$; $^1$H NMR (400 MHz, $CDCl_3$) δ: 9.53 (d, 1H), 8.84 (d, 1H), 7.82-7.69 (m, 1H), 7.64-7.52 (m, 2H), 7.50-7.40 (m, 1H), 4.58-4.42 (m, 2H), 1.50-1.45 (m, 3H).

Step 3: Preparation of Compound **5**

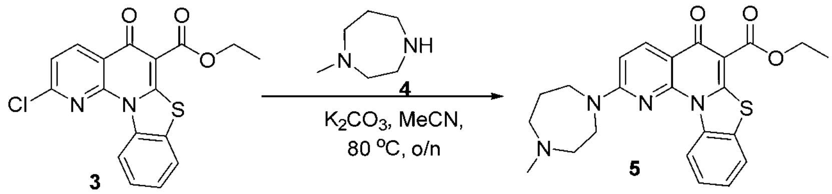


3 5

Compound **3** (5 g, 13.9 mmol, 1.0 eq.) and potassium carbonate (9.6 g, 69.5 mmol, 5.0 eq.) were added to acetonitrile (80 mL). Then N-methylhomopiperazine (3.2 g, 27.9 mmol, 2.0 eq.) was added to the system. Under nitrogen protection, the reaction mixture reacted overnight at 80°C. Once cooled to room temperature, the reaction mixture was filtered directly, and the filter cake was washed with water and methanol, and then dried to obtain Compound **5** (4.4 g, a light yellow solid, 72% yield). LC-MS (ESI): m/z 437.2 $[M+1]^+$; $^1$H NMR (400 MHz, DMSO-$d_6$) δ: 9.23 (d, 1H), 8.15 (d, 1H), 7.91-7.86 (m, 1H), 7.46-7.32 (m, 2H), 6.84 (d, 1H), 4.28 (q, 2H), 3.90-3.50 (m, 4H), 2.80-2.60 (m, 2H), 2.50-2.40 (m, 2H), 2.30-2.15 (m, 3H), 2.00-1.85 (m, 2H), 1.29 (t, 3H).

Step 4: Preparation of Compound **6**

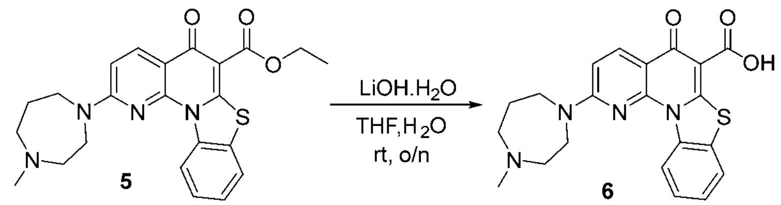


5 6

Compound **5** (4.45 g, 10.2 mmol, 1.0 eq.) was dissolved in tetrahydrofuran (30 mL) and water (30 mL), and lithium hydroxide monohydrate (1.23 g, 30.6 mmol, 3.0 eq.) was added to the system. The reaction mixture reacted overnight at room temperature, and then filtered directly. The filter cake was washed with ethyl acetate and dried under vacuum to obtain Compound **6** (4.15 g, light yellow solid, 98% yield). LC-MS (ESI): m/z 409.1 $[M+1]^+$; $^1$H NMR (400 MHz, DMSO-$d_6$) δ: 9.40 (d, 1H), 8.36 (d, 1H), 7.87 (d, 1H), 7.50-7.35 (m, 2H), 7.10-6.90 (m, 1H), 4.10-3.50 (m, 4H), 2.75-2.65 (m, 2H), 2.50-2.40 (m, 2H), 2.24 (s, 3H), 2.00-1.90 (m, 2H).

Step 5: Preparation of Compound **C2**

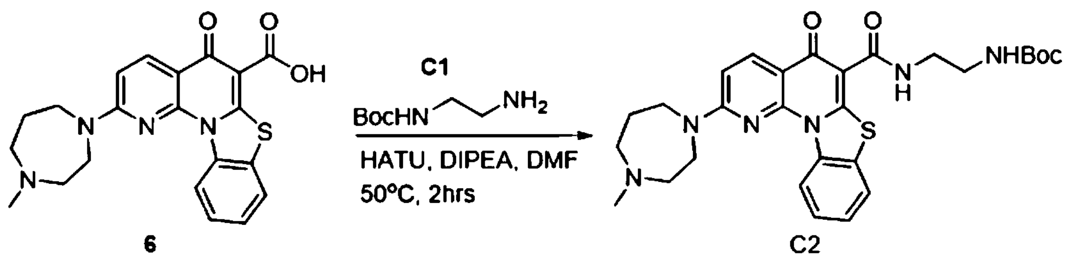


6 C2

Compound **6** (0.5 g, 1.23 mmol, 1.0 eq.) was dissolved in N,N-dimethylformamide (10 mL), and 2-(7-azabenzotriazol-1-yl)-1,1,3,3-tetramethyluronium hexafluorophosphate (HATU, 0.7 g, 1.84 mmol, 1.5 eq.) and N,N-diisopropylethylamine (DIEA, 0.64 mL, 3.68 mmol, 3.0 eq.) were added thereto. The reaction mixture reacted at 50°C under nitrogen protection for half an hour. Then Compound C1 (0.29 g, 1.84 mmol, 1.5 eq.) was added, and the reaction mixture was allowed to continue reacting at 50°C for 2 hours. The reaction mixture was spin dried to obtain a residue. The residue was slurried with methanol and filtered, and the filter cake was washed and dried to obtain Compound **C2** (500 mg, a yellow solid, 74.2% yield).

Step 6: Preparation of Compound **C3**

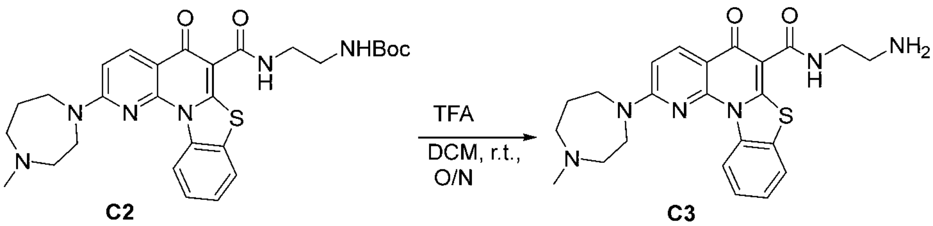


C2 C3

Compound **C2** (0.29 g, 0.53 mmol, 1.0 eq.) was dissolved in a mixed solution (DCM:TFA = 1:1, 6 mL). The reaction was conducted overnight at room temperature. The liquid in the reaction mixture was removed under reduced pressure to obtain Compound **C3** (238 mg, a yellow solid, 89.6% yield).

Step 7: Preparation of Compound **C**

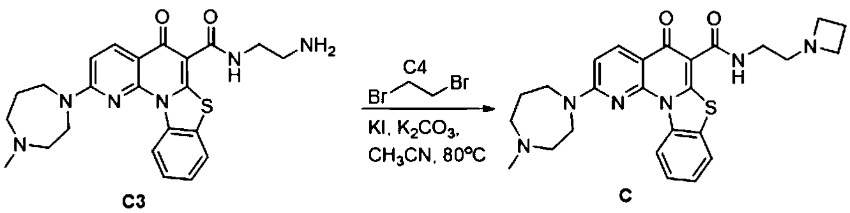


C3 C

Compound **C3** (0.238 g, 0.53 mmol, 1.0 eq.) was dissolved in acetonitrile (6 mL), and Compound **C4** (0.7 g, 1.59 mmol, 3.0 eq.), potassium carbonate (220 mg, 1.59 mmol, 3.0 eq.), and potassium iodide (26.6 mg, 0.16 mmol, 0.3 eq.) were added thereto. The mixture was heated to 80°C and reacted overnight. The reaction mixture was concentrated directly and subjected to preparative separation and purification to obtain Compound **C** (21 mg, a light yellow solid, 8.1% yield). LC-MS (ESI): m/z 491.4 $[M+1]^+$, $^1H$ NMR (400 MHz, $CD_3OD$) δ 9.55 - 9.45 (m, 1H), 8.55 - 8.45 (m, 1H), 7.95 - 7.85 (m, 1H), 7.60 - 7.45 (m, 2H), 7.10 - 7.00 (m, 1H), 4.30 - 4.15 (m, 4H), 4.10 - 4.00 (m, 2H), 3.98 - 3.88 (m, 2H), 3.70 - 3.60 (m, 2H), 3.48 - 3.40 (m, 3H), 3.18 - 3.12 (m, 2H), 3.00 - 2.92 (m, 2H), 2.61 (s, 3H), 2.54 - 2.45 (m, 2H), 2.35 - 2.25 (m, 2H).

Example 2: Preparation of Compound D

Steps 1 to 4 were the same as those in Example 1, and the prepared Compound **6** was for later use.

Step 5: Preparation of Compound **D**

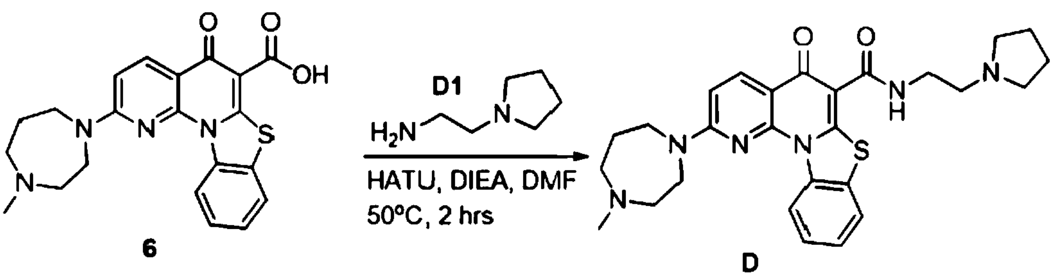


6 D

Compound **6** (0.1 g, 0.24 mmol, 1.0 eq.) was dissolved in N,N-dimethylformamide (3 mL), and 2-(7-azabenzotriazol-1-yl)-1,1,3,3-tetramethyluronium hexafluorophosphate (0.14 g, 0.37 mmol, 1.5 eq.) and N,N-diisopropylethylamine (0.13 mL, 0.73 mmol, 3.0 eq.) were added thereto. The reaction mixture reacted at 50°C under nitrogen protection for half an hour. Then 1-pyrrolidineethanamine (0.042 g, 0.37 mmol, 1.5 eq.) was added, and the reaction mixture was allowed

to continue reacting at 50°C for 2 hours. The liquid in the reaction mixture was removed under reduced pressure, and the residue was slurried with methanol, filtered, and dried to obtain Compound **D** (74 mg, a yellow solid, 60% yield). LC-MS (ESI): m/z 505.2 $[M+1]^+$, $^1$H NMR (400 MHz, $CD_3OD$) δ 9.40 - 9.30 (m, 1H), 8.45 - 8.35 (m, 1H), 7.90 - 7.80 (m, 1H),7.60 - 7.40 (m, 2H), 7.05 - 6.95 (m, 1H), 4.20 - 4.10 m, 2H), 3.90 -3.80 (m, 4H), 3.55 - 3.45 (m, 8H), 3.40 - 3.30 (m, 2H), 2.87 (s, 3H), 2.40 - 2.30 (m, 2H), 2.15 - 2.05 (m, 4H).

Example 3: Preparation of Compound E

Steps 1 to 4 were the same as those in Example 1, and the prepared Compound 6 was for later use.

Step 5: Preparation of Compound **E**

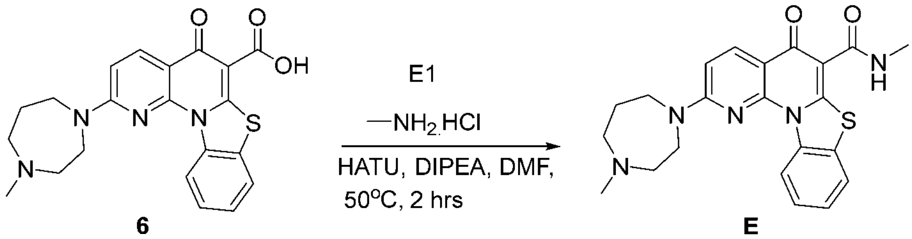


6 E

Compound 6 (0.15 g, 0.37 mmol, 1.0 eq.) was dissolved in N,N-dimethylformamide (3 mL), and 2-(7-azabenzotriazol-1-yl)-1,1,3,3-tetramethyluronium hexafluorophosphate (0.21 g, 0.56 mmol, 1.5 eq.) and N,N-diisopropylethylamine (0.2 mL, 1.11 mmol, 3.0 eq.) were added thereto. The reaction mixture reacted at 50°C under nitrogen protection for half an hour. Then methylamine hydrochloride (0.032 g, 0.48 mmol, 1.3 eq.) was added, and the reaction mixture was allowed to continue reacting at 50°C for 2 hours. The reaction mixture was concentrated directly to remove DMF, and methanol (5 mL) was added. The reaction mixture was stirred at room temperature for 1 hour, and filtered. The filter cake was washed with methanol and dried under vacuum to obtain Compound **E** (0.099 g, a light yellow solid, 63.8% yield). LC-MS (ESI): m/z 422.2 $[M+1]^+$, $^1$H NMR (400 MHz, DMSO-$d_6$) δ10.30 - 10.20 (m, 1H), 9.70 - 9.45 (m, 1 H),9.35 - 9.20 (m, 1H), 8.40 - 8.30 (m, 1H), 8.05 - 7.95 (m, 1H), 7.65 - 7.50 (m, 1H), 7.50 - 7.35 (m, 1H), 7.15 - 7.00 (m, 1H), 4.00 - 3.55 (m, 4H), 3.55 - 3.30 (m, 4H), 2.90 - 2.85 (m, 3H), 2.83 (s, 3H), 2.30 - 2.20 (m, 2H).

Example 4: Preparation of Compound F

Steps 1 to 4 were the same as those in Example 1, and the prepared Compound **6** was for later use.

Step 5: Preparation of Compound **F**

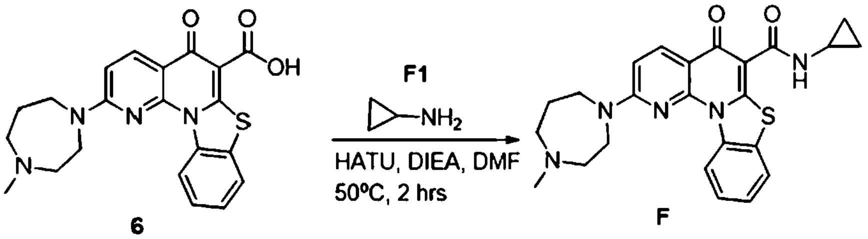


**6** **F**

Compound **6** (0.15 g, 0.37 mmol, 1.0 eq.) was dissolved in N,N-dimethylformamide (3 mL), and 2-(7-azabenzotriazol-1-yl)-1,1,3,3-tetramethyluronium hexafluorophosphate (0.21 g, 0.56 mmol, 1.5 eq.) and N,N-diisopropylethylamine (0.2 mL, 1.11 mmol, 3.0 eq.) were added thereto. The reaction mixture reacted at 50°C under nitrogen protection for half an hour. Then cyclopropylamine (0.027 g, 0.48 mmol, 1.3 eq.) was added, and the reaction mixture allowed to continue reacting at 50°C for 2 hours. N,N-dimethylformamide in the reaction mixture was removed under reduced pressure, and methanol (5 mL) was added. The mixture was stirred at room temperature for 1 hour, and filtered. The filter cake was washed with methanol and dried under vacuum to obtain Compound **F** (0.084 g, a light yellow solid, 51.2% yield). LC-MS

(ESI): m/z 448.2 $[M+1]^+$, $^1H$ NMR (400 MHz, DMSO-$d_6$) $\delta$ 10.50 - 10.40 (m, 1H), 9.70 - 9.60 (m, 1H), 9.30 - 9.15 (m, 1H), 8.35 - 8.25 (m, 1H), 8.05 - 7.95 (m, 1H), 7.60 - 7.40 (m, 2H), 7.10 - 7.00 (m, 1H), 3.95 - 3.55 (m, 4H), 3.50 - 3.30 (m, 4H), 2.95 - 2.75 (m, 4H), 2.30 - 2.20 (m, 2H), 0.80 - 0.70 (m, 2H), 0.60 - 0.50 (m, 2H).

Example 5: Preparation of Compound G-1

Steps 1 to 4 were the same as those in Example 1, and the prepared Compound 6 was for subsequent use.

Step 5: Preparation of Compound **G3**

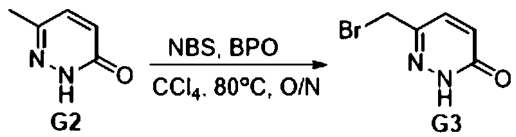


**G2** **G3**

Compound **G2** (0.5 g, 4.54 mmol, 1.0 eq.) was added to carbon tetrachloride (10 mL), followed by the addition of N-bromosuccinimide (NBS, 849 mg, 4.77 mmol, 1.05 eq.) and benzoyl peroxide (BPO, 220 mg, 0.98 mmol, 0.2 eq.) to the system. The reaction was carried out overnight at 80°C under nitrogen protection. The reaction mixture was washed with a large amount of water to remove metabolites of NBS, and then extracted with dichloromethane. The organic phase was directly concentrated and separated by column chromatography to obtain Compound **G3** (0.38 g, an off-white solid, 44% yield). $^1H$ NMR (400 MHz, DMSO-$d_6$) $\delta$ 11.02 (s, 1H), 7.49 (d, 1H), 6.89 (d, 1H), 4.51 (s, 2H).

Step 6: Preparation of Compound **G1**

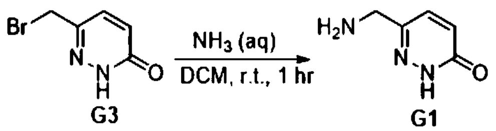


**G3** **G1**

Compound **G3** (0.31 g, 1.0 eq.) was dissolved in dichloromethane (10 mL), and ammonia gas was injected through the solution at an appropriate rate for 3 minutes. The reaction mixture reacted at room temperature for 1 hour and directly concentrated to obtain Compound **G1** (0.25 g, a light yellow solid; the crude product was used directly in the subsequent step). $^1H$ NMR (400 MHz, DMSO-$d_6$) $\delta$ 7.45 (dd, $J$ = 14.0, 9.8 Hz, 3H), 6.93 (d, $J$ = 9.8 Hz, 1H), 3.98 (s, 2H).

Step 7: Preparation of Compound **G-1**

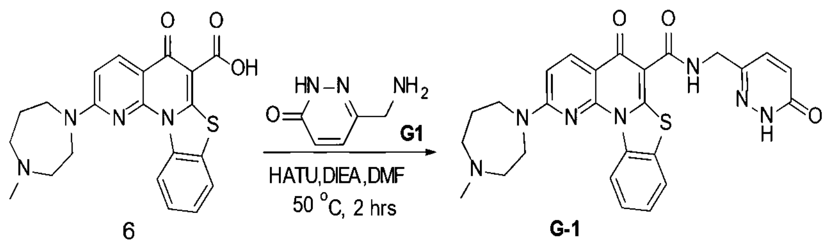


6 **G-1**

Compound **6** (0.15 g, 0.37 mmol, 1.0 eq.) was dissolved in N,N-dimethylformamide (6 mL), and 2-(7-azabenzotriazol-1-yl)-1,1,3,3-tetramethyluronium hexafluorophosphate (0.211 g, 0.56 mmol, 1.5 eq.) and N,N-diisopropylethylamine (0.19 mL, 1.1 mmol, 3.0 eq.) were added thereto. The reaction mixture reacted at 50°C for half an hour under nitrogen protection, then Compound **G1** (0.137 g, 1.1 mmol, 3.0 eq.) was added, and the reaction mixture was allowed to continue reacting at 50°C for 2 hours. The reaction mixture was directly concentrated to remove N,N-dimethylformamide, and methanol (3 mL) was added. The reaction mixture was stirred overnight at room temperature, and filtered. The filter cake was washed with methanol, and purified to obtain Compound **G-1** (0.04 g, a light yellow solid, 21.1% yield). LC-MS (ESI):

m/z 516.2 [M+1]+, $^1$H NMR (400 MHz, DMSO-$d_6$) δ 12.91 (s, 1H), 11.00 - 10.90 (m, 1H), 9.45 - 9.35 (m, 1H), 8.40 - 8.30 (m, 1H), 8.05 - 7.95 (m, 1H), 7.60 - 7.50 (m, 1H), 7.50 - 7.40 (m, 2H), 7.05 - 6.95 (m, 1H), 6.90 - 6.80 (m, 1H), 4.55 - 4.45 (m, 2H), 3.96 - 3.38 (m, 4H), 2.95 - 2.75 (m, 2H), 2.70 - 2.55 (m, 2H), 2.36 (s, 3H), 2.10 - 1.95 (m, 2H).

Example 6: Preparation of Compound G-2

Step 1: Preparation of Compound 2

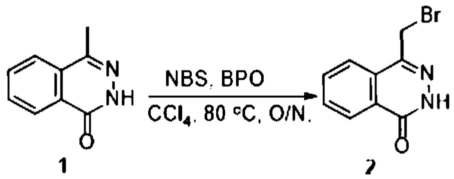


1 2

Compound **1** (1.0 g, 6.2 mmol, 1.0 eq.) was added to carbon tetrachloride (12 mL), followed by the addition of N-bromosuccinimide (NBS, 1.16 g, 6.5 mmol, 1.05 eq.), azobisisobutyronitrile (AIBN, 204 mg, 1.2 mmol, 0.2 eq.), and benzoyl peroxide (BPO, 300 mg, 1.2 mmol, 0.2 eq.) to the system. The reaction was carried out overnight at 80°C under nitrogen protection. The reaction mixture was concentrated and separated by column chromatography to obtain Compound **2** (0.20 g, an off-white solid, 13.4% yield). $^1$H NMR (400 MHz, DMSO-$d_6$) δ 12.78 (s, 1H), 8.25 (dd, *J* = 8.0, 1.3 Hz, 1H), 8.06 (d, *J* = 8.1 Hz, 1H), 7.97 (ddd, *J* = 8.1, 7.1, 1.4 Hz, 1H), 7.86 (td, *J* = 7.6, 1.2 Hz, 1H), 4.93 (s, 2H).

Step 2: Preparation of Compound 3

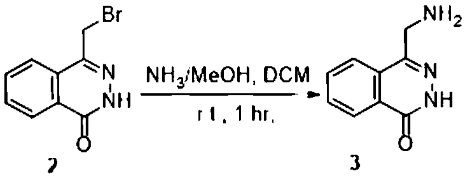


2 3

Compound **2** (0.5 g, 2.1 mmol, 1.0 eq.) was dissolved in dichloromethane (10 mL), and ammonia/methanol (7M, 5 mL) was added. The resulting mixture reacted at room temperature for 1 hour. The reaction mixture was concentrated and purified by reversed-phase column chromatography to obtain Compound **3** (0.22 g, a light yellow solid, 68.3% yield). $^1$H NMR (400 MHz, DMSO--$d_6$) δ 12.91 (s, 1H), 8.27 (d, J = 7.8 Hz, 1H), 8.00 - 7.94 (m, 2H), 7.90 (td, J = 5.6, 2.7 Hz, 1H), 4.45 (s, 2H).

Step 3: Preparation of Compound **G-2**

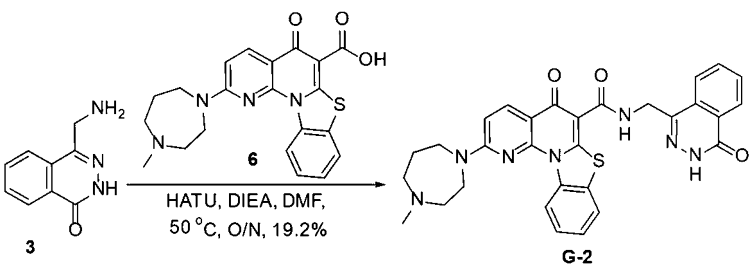


3 6 G-2

Compound **6** (500 mg, 1.2 mmol, 1.0 eq.) was dissolved in N,N-dimethylformamide (8 mL), and 2-(7-azabenzotriazol-1-yl)-1,1,3,3-tetramethyluronium hexafluorophosphate (684 mg, 1.8 mmol, 1.5 eq.) and N,N-diisopropylethylamine (0.62 mL, 3.6 mmol, 3.0 eq.) were added thereto. The reaction mixture reacted at 50°C for half an hour under nitrogen protection. Then Compound **3** (525 mg, 3.0 mmol, 2.5 eq.) was added, and the reaction mixture was allowed to continue

reacting at 50°C for 16 hours. The reaction mixture was concentrated to remove N,N-dimethylformamide, and methanol (10 mL) was added. The mixture was stirred overnight at room temperature, and filtered. The filter cake was washed with methanol, concentrated, and purified to obtain Compound **G-2** (132.9 mg, a light yellow solid, 19.2% yield). LC-MS (ESI): m/z 566.5 $[M+1]^+$, $^1H$ NMR (400 MHz, DMSO-$d_6$) δ 12.67 (s, 1H), 11.15 - 11.00 (m, 1H), 9.45 - 9.30 (m, 1H), 8.30 - 8.20 (m, 2H), 8.10 - 8.00 (m, 2H), 8.00 - 7.90 (m, 2H), 7.90 - 7.80 (m, 1H), 7.60 - 7.35 (m, 2H), 7.00 - 6.90 (m, 1H), 4.90 - 4.75 (m, 2H), 4-00 - 3.55 (m, 4H), 3.40 - 3.30 (m, 2H), 2.80 - 2.60 (m, 2H), 2.24 (s, 3H), 2.05 - 1.90 (m, 2H).

Example 7: Biological Evaluation

The following test examples further describe and explain the present application, but these examples are not intended to limit the scope of the present application.

Experimental methods not specified with particular conditions in the test examples of the present application are generally performed under conventional conditions, or under the conditions recommended by the commodity manufacturer. Reagents not specified with particular sources are commercially available conventional reagents.

Test Example 1. Inhibition of Compounds on Cancer Cells

Testing Method for Detecting Cell Activity

(1) Compound Treatment

a) Preparation of Stock Solutions of Compounds
Test compounds were dissolved in DMSO at a concentration of 10 mM or 5 mM, and Olaparib was dissolved in DMSO at a concentration of 10 mM.
b) Compound Storage
All compounds dissolved in DMSO were stored in a desiccator at room temperature for short-term storage and transferred to a -20°C refrigerator for long-term storage.
c) Preparation of Working Concentrations of Compounds

Test compounds were serially diluted in DMSO at a 3-fold gradient to prepare 10 concentration gradients, starting from 10 μM.

Olaparib was serially diluted in DMSO at a 3-fold gradient to prepare 10 concentration gradients, starting from 10 μM.

The 384-well plate loaded with the compounds was centrifuged at 1000 rpm for 1 minute.

(2) Activity Assay

a) Cells were added to the culture medium and incubated in a cell culture incubator at 37°C with 5% $CO_2$.
b) 40 μL of BRCA1- or BRCA2-deficient cells were added to a 384-well plate and incubated overnight at 37°C with 5% $CO_2$.
c) The test compounds were prepared at different concentration gradients on the master plate, and 80 nL of the test compounds and Olaparib were taken from the master plate and added to the 384-well plate containing cells, and incubated at 37°C with 5% $CO_2$ for 6 days.
d) CellTiterGlo (30 μL per well) was added to the 384-well plate, and after incubation at room temperature in the dark for 30 minutes, measurement was performed using an Envision plate reader.

(3) Data Processing

a) GraphPad Prism software was used to calculate the ICso and plot the activity curves as a function of concentration.

b) The inhibition of cell activity by the compounds was calculated using the following formula:

% Inhibition = [1 - (Cmpd-Blank) / (DMSO-Blank)] * 100

Test Results:

Table 1. Study on the Activity Inhibition of Quinolone Analogs Against BRCA2 Gene Knockout DLD1 Cancer Cells

| ID# | Structures | $IC_{50}$(nM) DLD1 $BRCA2^{-/-}$ |
|---|---|---|
| **Olaparib** | 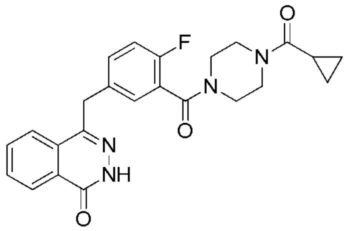 | 489.8 |
| **CX-5461** | 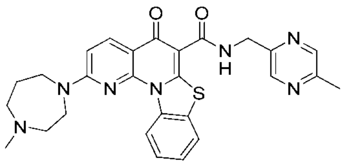 | **43.9** |
| **Compound C** | 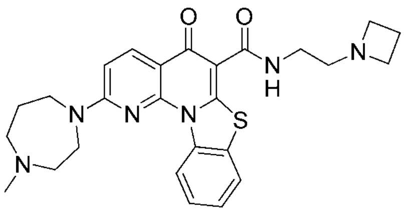 | 4.8 |
| **Compound D** | 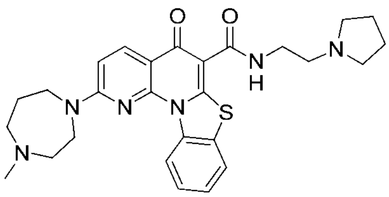 | 4.3 |
| **Compound E** | 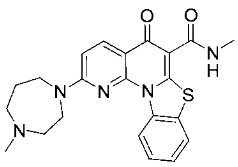 | 11.0 |
| **Compound F** | 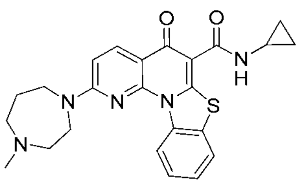 | 17.2 |
| **Compound G-1** | 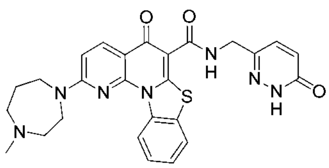 | 32.7 |

The compounds shown in the above table exhibit significantly higher inhibition of the activity of BRCA2 gene knockout DLD1 cancer cells compared to the PARP inhibitor Olaparib and CX-5461.

Table 2. Study on the Activity Inhibition of Quinolone Analogs Against BRCA1 Gene-Deficient Ovarian **Cancer Cells UWB1.289**

| ID# | Structures | $IC_{50}$ (nM) UWB1.289 Cells |
|---|---|---|
| **Olaparib** | 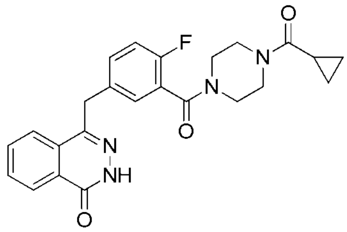 | 716 |
| **Compound** (CX-5461) | 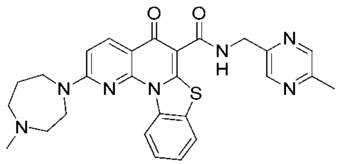 | 91.6 |
| **Compound C** | 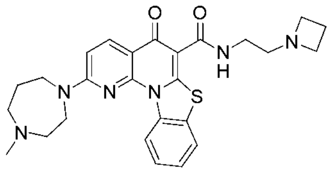 | 4.9 |
| **Compound D** | 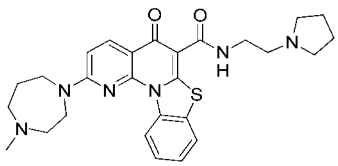 | 5.8 |
| **Compound E** | 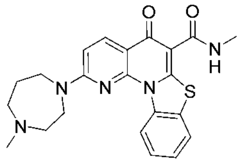 | 39.8 |
| **Compound F** | 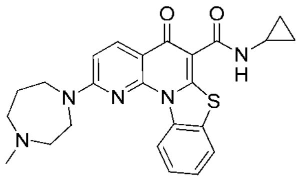 | 40.6 |
| **Compound G-1** | 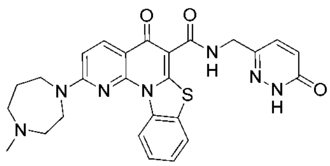 | 40.4 |

The studies on the activity of BRCA1 gene-deficient ovarian cancer cells UWB1.289 revealed that Compounds C, D, E, F and G-1 exhibit a stronger ability to kill BRCA1-deficient cancer cells than the control compounds Olaparib and CX-5461, with a significantly lower $IC_{50}$ than Olaparib and CX-5461.

Table 3. Study on the Activity Inhibition of Quinolone Analogs Against **BRCA1 Gene-Deficient Ovarian Cancer MDA-MB-436**

| ID# | Structures | $IC_{50}$(nM) MDA-MB-436细胞 |
|---|---|---|
| **Olaparib** | 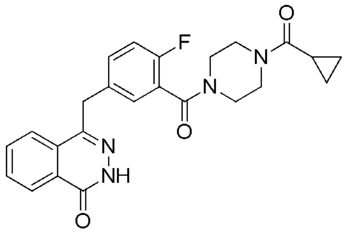 | 240 |
| **Compound** (CX-5461) | 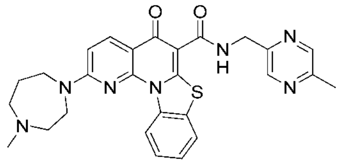 | 10.0 |
| **Compound E** | 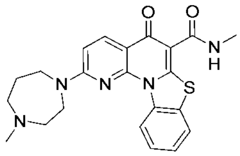 | 7.7 |

The studies on the activity of BRCA1 gene-deficient ovarian cancer cells MDA-MB-436 revealed that Compound E exhibits a stronger ability to kill BRCA1-deficient cancer cells than the control compounds Olaparib and CX-5461, with a significantly lower $IC_{50}$ than Olaparib and CX-5461.

Test Example 2. hERG Cardiotoxicity Assay of Compounds

Assay Method:

1) The hERG stably expressed HEK 293 cell line (Cat# K1236) was purchased from Invitrogen. Cells were cultured in a medium consisting of 85% DMEM, 10% FBS, 0.1 mM NEAA, 25 mM HEPES, 100 U/mL penicillin-streptomycin, 5 μg/mL Blasticidin, and 400 μg/mL Geneticin. Cells were passaged approximately three times a week using TrypLE™ Express and maintained at ~40% to ~80% confluency. Before assay, cells were placed on coverslips with $5\times10^5$ cells per 6-cm cell culture dish and induced with 1 μg/mL doxycycline for 48 hours.
2) The coverslip was removed from the cell culture dish and placed on the microscope stage. A suitable cell was located using a 10× objective. The tip of the electrode was located under the microscope using the 10× objective, and the focus was adjusted to the plane of the cell. Once the tip was in focus, the coarse control of the manipulator was used to advance the electrode downward toward the cell while moving the objective to keep the tip in focus. When directly above the cell, the objective was switched to 40× objective, and the fine control of the manipulator was used to approach the cell surface in small steps.
3) A blank control was first performed to establish a baseline. Once the hERG current was found to be stable for 5 minutes, the working solution was applied. In the presence of the test compounds, the hERG current was recorded for approximately 5 minutes to reach a steady state, followed by capturing 5 scans. For dose-response testing, 5 doses of the test compounds were cumulatively applied to the cells from low to high concentrations. The positive control, dofetilide at a concentration of 150 nM, was applied to each cell, and the hERG current was measured in the test compounds at the highest concentration as a negative control to normalize the percentage of inhibition. To ensure good performance of the cultured cells and operations, cells from the same batch were also tested with 5 doses of dofetilide.

Table 4. hERG Toxicity Assay Results

| Test Compounds | hERG $IC_{50}$ [μM] |
|---|---|
| CX-5461 | 5.062 |
| Compound G-1 | >30 |
| Compound G-2 | >10 |

Test Results: The $IC_{50}$ values of the compounds listed in Table 4 in the hERG toxicity assay are higher than that of CX-5461, indicating an improvement in cardiotoxicity compared to CX-5461.

Test Example 3. Inhibition of Viral DNA Replication by Compounds

A segment of viral DNA containing a quadruplex DNA sequence was used as a template, and the primer was fluorescently labeled and annealed to the 3'-end of the template DNA. Taq DNA polymerase was added to the system to synthesize a complementary strand using the fluorescently labeled primer. If DNA synthesis was not inhibited, a full-length copy of the template would be synthesized. If the added compound bound non-specifically to the doublestranded DNA, the full-length product would decrease, and PCR products of different short lengths would be formed. If the compound specifically bound to the quadruplex DNA in the viral DNA sequence, DNA replication would only stop at the quadruplex DNA region. PCR products were detected by DNA electrophoresis to determine whether the compound inhibits viral DNA replication compared to the vehicle control.

The experimental results showed that the $IC_{50}$ of the compound of Formula (I) for inhibiting viral DNA replication was 0.2-10 μM. Therefore, the compound of Formula (I) has strong inhibitory effects on the replication of viral DNAs containing quadruplex DNA structures.

Test Example 4. Determination of Antiviral Cell-Protective Activity of Compounds

The alamarBlue® (Invitrogen) kit was used to detect the toxic effects of the drugs and the viruses on cells.

Experimental Procedures: MRC-5 fibroblasts (ATCC) were seeded into 96-well cell culture plates and allowed to adhere before further use. The drug was diluted in DMEM medium starting from 2 times the highest test concentration, followed by 3-fold serial dilutions across 8 gradients. The drug was added to the cells, and the cells were cultured in a $CO_2$ incubator at 37°C. After 48 hours of drug treatment, the cytopathic effects caused by the drug were observed under a microscope, and alamar Blue was added to detect cell viability. The toxicity of the virus to cells was inversely proportional to cell activity, and was reflected by cell activity. The experiment set up blank control wells (normal cells), virus control wells (virus-infected cells without drug), drug wells (normal uninfected cells treated with drug), and virus + drug wells (virus-infected cells treated with the drug).

The experiment showed that when the concentrations of the compounds of Formula (I) were approximately 0.05-5 μM, the compounds exhibited a protective effect against the decrease in cell activity caused by viral infection.

Test Example 5. Inhibition of Multiple Sclerosis by Compounds of Formula (I)

The autoimmune encephalomyelitis (EAE) model was established as follows:
C57BL/6 male mice, aged 6-8 weeks, were divided into groups with 6 mice per group:

Group 1: EAE + vehicle;
Group 2: EAE + PRN2246;
Group 3: EAE + test compound.

The administration frequency was once a day, and the administration cycle lasted for 21 days. MOG35-55 was diluted with PBS, and the diluted solution was mixed with the complete Freund's adjuvant. The mixture was emulsified into a water-in-oil state using a glass syringe to form an antigen emulsion for EAE induction. On the day of immunization and the second day, the mice were intraperitoneally injected with pertussis toxin to induce the establishment of the mouse autoimmune encephalomyelitis model.

Neurological function scoring criteria:

The commonly used 0-5 grade scoring scale was adopted, where:

Grade 0: No disease onset;
Grade 1: Decreased tail tension or tail paralysis in mice;
Grade 2: Tail paralysis and hindlimb weakness in mice;
Grade 3: Hindlimb palsy in mice;
Grade 4: Quadriplegia in mice, with inability to reset when manually turned over;
Grade 5: Near-death state or death after disease onset.

Scoring frequency: Starting from day 11, scoring was performed once a day for a total of 10 days. Body weight measurement: twice per week.

The experiment showed that when the concentrations of the compounds of Formula (I) were 0.15-15 μM, the compounds exerted a delaying effect on the progression of multiple sclerosis.

The specific examples of the present application have been described above; however, it should be understood that the present application is not limited to the above-specified embodiments. Those skilled in the art can make various variations and modifications without departing from the concept of the present application, and all such variations and modifications fall within the protection scope of the present application.

## Claims

1. A compound of Formula (I), or a pharmaceutically acceptable salt, stereoisomer, racemate, deuterated compound, or solvate thereof:

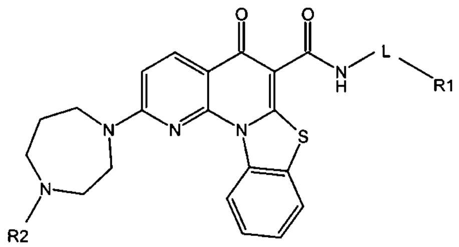

Formula (I)

wherein:

R1 is selected from C1-C6 linear or branched alkyl, C5-C10 aryl, C5-C10 heteroaryl, C3-C8 cycloalkyl, or C3-C8 heterocycloalkyl, wherein the C1-C6 linear or branched alkyl, the C5-C10 aryl, the C5-C10 heteroaryl, the C3-C8 cycloalkyl, and the C3-C8 heterocycloalkyl are each independently optionally substituted with a substituent or unsubstituted;
L is $-(CH_2)n-$, where n = 0-6;
R2 is selected from H, deuterium, C1-C4 alkyl, or C1-C4 alkyl substituted with one or more deuterium atoms;
the substituent is independently selected from oxo, C1-C4 alkyl, hydroxy(C1-C4 alkyl), halogen, cyano, hydroxyl, C1-C4 alkylamino, C1-C4 alkoxy, or halo(C1-C4 alkyl)); and
the heteroaryl is 5- to 10-membered heteroaryl containing one or more types of heteroatoms selected from N, O, or S with a number of the heteroatoms from 1 to 3.

2. The compound of Formula (I), or the pharmaceutically acceptable salt, stereoisomer, racemate, deuterated compound, or solvate thereof according to claim 1, wherein:

R1 is selected from C1-C6 linear or branched alkyl, C5-C10 heteroaryl, C3-C8 cycloalkyl, or C3-C8 heterocycloalkyl, wherein the C1-C6 linear or branched alkyl, the C5-C10 heteroaryl, the C3-C8 cycloalkyl, and the C3-C8 heterocycloalkyl are each independently optionally unsubstituted or substituted with a substituent, and the substituent is independently selected from oxo, C1-C4 alkyl, hydroxy(C1-C4 alkyl), halogen, cyano, hydroxyl, C1-C4 alkylamino, C1-C4 alkoxy, or halo(C1-C4 alkyl), and the heteroaryl is 5-to 10-membered heteroaryl containing one or more types of heteroatoms selected from N, O or S with a number of the heteroatoms from 1 to 3; or
R1 is selected from methyl,

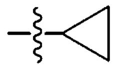, 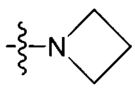, 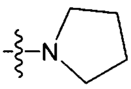, 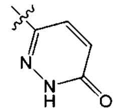,

or

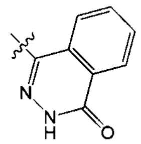.

3. The compound of Formula (I), or the pharmaceutically acceptable salt, stereoisomer, racemate, deuterated compound, or solvate thereof according to claim 1 or 2, wherein L is $-(CH_2)_n-$, where n = 0-3; or L is $-(CH_2)_n-$, where n = 0-2; or L is $-CH_2-$ or $-CH_2CH_2-$; or L is $-(CH_2)_n-$, where n = 0.

4. The compound of Formula (I), or the pharmaceutically acceptable salt, stereoisomer, racemate, deuterated compound, or solvate thereof according to any one of claims 1 to 3, wherein R2 is selected from C1-C4 alkyl; or R2 is methyl.

5. The compound of Formula (I), or the pharmaceutically acceptable salt, stereoisomer, racemate, deuterated compound, or solvate thereof according to claim 1, wherein the compound is selected from the following structural formulas:

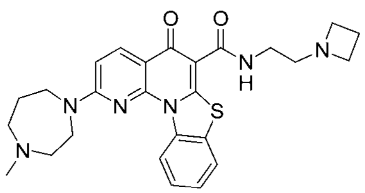 C, 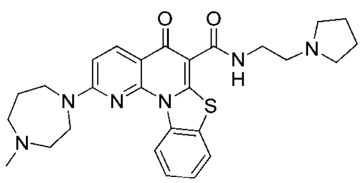 D, 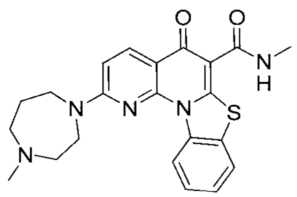 E,

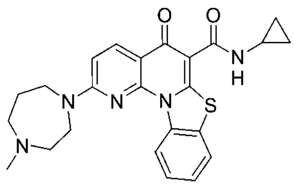 F, 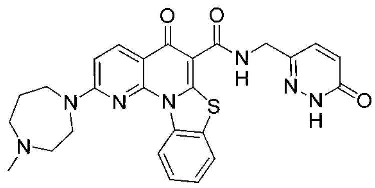 G-1, 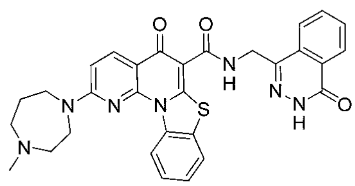 G-2.

6. A pharmaceutical composition comprising an effective amount of the compound of Formula (I), or the pharmaceutically acceptable salt, stereoisomer, racemate, deuterated compound, or solvate thereof according to any one of claims 1 to 5, and a pharmaceutically acceptable excipient.

7. Use of the compound of Formula (I), or the pharmaceutically acceptable salt, stereoisomer, racemate, deuterated compound, or solvate thereof according to any one of claims 1 to 5, or the pharmaceutical composition according to claim 6 in the preparation of a medicament for the treatment of tumors, autoimmune diseases, or diseases caused by bacteria, fungi or viruses.

8. The use according to claim 7, wherein the tumors are a cancer, wherein the cancer is a cancer with BRCA-1 or BRCA-2 mutation (homozygous or heterozygous mutation), a homologous recombination repair-deficient cancer, a non-homologous end joining (NHEJ) repair-deficient cancer, or other DNA damage repair-deficient cancers, or a cancer with overexpression of c-Myc, N-Myc or L-Myc or a cancer with an abnormal chromosome number.

9. The use according to claim 7, wherein the tumors are a cancer, wherein the cancer is breast cancer, ovarian cancer, endometrial cancer, cervical cancer, oral cancer, pancreatic cancer, prostate cancer, brain cancer, lung cancer, liver cancer, leukemia, lymphoma, myeloma, multiple myeloma, skin cancer, peritoneal cancer, colorectal cancer,

glioblastoma, melanoma, osteosarcoma, cervical cancer, Ewing's sarcoma, lymph node cancer, gastrointestinal malignancy, head and neck cancer, kidney cancer, or cardiac cancer.

**10.** The use according to claim 7, wherein the autoimmune diseases are multiple sclerosis.

**11.** The use according to claim 7, wherein the viruses are selected from hepatitis B virus, hepatitis C virus, rhinovirus, varicella-zoster virus, herpes simplex virus, cytomegalovirus, poxvirus, encephalitis virus, hantavirus, arbovirus, human papillomavirus, Sironi virus, human immunodeficiency virus, influenza virus, Epstein-Barr (EB) virus, respiratory syncytial virus, or coronavirus.

**12.** Use of the compound of Formula (I), or the pharmaceutically acceptable salt, stereoisomer, racemate, deuterated compound, or solvate thereof according to any one of claims 1 to 5, or the pharmaceutical composition according to claim 6 in the preparation of a medicament for the treatment of diseases that are ameliorated or rendered resistant by the use of a PARP inhibitor, or use of the compound of Formula (I), or the pharmaceutically acceptable salt, stereoisomer, racemate, deuterated compound, or solvate thereof according to any one of claims 1 to 5, or the pharmaceutical composition according to claim 6 in combination with a PARP inhibitor drug, wherein the PARP inhibitor drug used in combination is selected from Olaparib, Niraparib, Rucaparib, Talazoparib, Fluzopari, or Pamipari.

**13.** Use of the compound of Formula (I), or the pharmaceutically acceptable salt, stereoisomer, racemate, deuterated compound, or solvate thereof according to any one of claims 1 to 5, or the pharmaceutical composition according to claim 6, in combination with topoisomerase I and topoisomerase II inhibitors, CHK1 inhibitors, CHK2 inhibitors, ATM inhibitors, ATR inhibitors, DNA-pK inhibitors, WEE1 inhibitors, DNA polymerase inhibitors, RNA polymerase inhibitors, DNA damage repair network targeting drugs, mTORC1/2 inhibitors, histone deacetylase inhibitors, proteasome inhibitors, RNA transcription inhibitors, mRNA translation inhibitors, PIM kinase, or p53 activators.

**14.** Use of the compound of Formula (I), or the pharmaceutically acceptable salt, stereoisomer, racemate, deuterated compound, or solvate thereof according to any one of claims 1 to 5, or the pharmaceutical composition according to claim 6 in the preparation of a medicament for use in combination with chemotherapy, radiotherapy, targeted therapy, immunotherapy, immune checkpoint inhibitor therapy, endocrine therapy, metabolic therapy or oncolytic virus therapy for a cancer, wherein the cancer is preferably breast cancer, ovarian cancer, endometrial cancer, cervical cancer, oral cancer, pancreatic cancer, prostate cancer, lung cancer, liver cancer, brain cancer, leukemia, lymphoma, myeloma, skin cancer, peritoneal cancer, osteosarcoma, lymph node cancer, gastrointestinal malignancy, head and neck cancer, kidney cancer, or cardiac cancer.

## INTERNATIONAL SEARCH REPORT

International application No.
**PCT/CN2024/089126**

**A. CLASSIFICATION OF SUBJECT MATTER**

C07D513/14(2006.01)i； A61P35/00(2006.01)i； A61K31/551(2006.01)i

According to International Patent Classification (IPC) or to both national classification and IPC

**B. FIELDS SEARCHED**

Minimum documentation searched (classification system followed by classification symbols)

IPC: C07D,A61P,A61K

Documentation searched other than minimum documentation to the extent that such documents are included in the fields searched

Electronic data base consulted during the international search (name of data base and, where practicable, search terms used)

万方, WANFANG, CNKI, CNTXT, VEN, ENTXT, STNext-REGISTRY, CAPLUS, MARPAT: 信义核新, 喹诺酮, 四环喹诺酮, 癌, 肿瘤, 自身免疫性疾病, cancer, tumor, CX-5461, quinolone, tetracycloquinolone, autoimmune disease, STN-structure search

**C. DOCUMENTS CONSIDERED TO BE RELEVANT**

| Category* | Citation of document, with indication, where appropriate, of the relevant passages | Relevant to claim No. |
|---|---|---|
| A | CN 101888780 A (CYLENE PHARMACEUTICALS INC.) 17 November 2010 (2010-11-17) see entire document, in particular claim 19 | 1-11, 12 (in part), 14 |
| A | CN 115919873 A (XINYI HEXIN (BEIJING) BIOLOGICAL TECHNOLOGY CO., LTD.) 07 April 2023 (2023-04-07) see entire document | 1-11, 12 (in part), 14 |
| A | CN 108601789 A (SENHWA BIOSCIENCES, INC.) 28 September 2018 (2018-09-28) see entire document | 1-11, 12 (in part), 14 |
| A | WO 2008131134 A1 (CYLENE PHARMACEUTICALS INC. et al.) 30 October 2008 (2008-10-30) see entire document | 1-11, 12 (in part), 14 |
| A | CN 103533934 A (TEL HASHOMER MEDICAL RESEARCH INFRASTRUCTURE AND SERVICES LTD.) 22 January 2014 (2014-01-22) see entire document | 1-11, 12 (in part), 14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 July 2024** | **23 July 2024** |
| Name and mailing address of the ISA/CN<br>**China National Intellectual Property Administration (ISA/CN)<br>China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/089126**

**C. DOCUMENTS CONSIDERED TO BE RELEVANT**

| Category* | Citation of document, with indication, where appropriate, of the relevant passages | Relevant to claim No. |
|---|---|---|
| A | CN 114409681 A (XINYI HEXIN (BEIJING) BIOLOGICAL TECHNOLOGY CO., LTD.) 29 April 2022 (2022-04-29)<br>see entire document | 1-11, 12 (in part), 14 |
| A | WO 2015079411 A1 (TEL HASHOMER MEDICAL RES INFRASTRUCTURE & SERVICES LTD.) 04 June 2015 (2015-06-04)<br>see entire document | 1-11, 12 (in part), 14 |
| A | SULLIVAN Holli-Joi et al. "Molecular Dynamics Study on the Binding of an Anticancer DNA G-Quadruplex Stabilizer, CX-5461, to Human Telomeric, c-KIT1, and c-Myc G-Quadruplexes and a DNA Duplex"<br>*Journal of Chemical Information and Modeling,* Vol. vol. 60, 21 August 2020 (2020-08-21),<br>pp. 5203-5224 | 1-11, 12 (in part), 14 |

Form PCT/ISA/210 (second sheet) (July 2022)

**INTERNATIONAL SEARCH REPORT**

International application No.
**PCT/CN2024/089126**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12 (in part), 13**
because they relate to subject matter not required to be searched by this Authority, namely:

Part of the subject matter of claim 12 is "the combination with a PARP inhibitor drug", and the subject matter and claim 13 relate to a treatment method for a human or animal body, which falls within the cases set out in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/089126**

| Patent document cited in search report | | | Publication date (day/month/year) | Patent family member(s) | | | Publication date (day/month/year) |
|---|---|---|---|---|---|---|---|
| CN | 101888780 | A | 17 November 2010 | RU | 2010117225 | A | 10 November 2011 |
| | | | | RU | 2549895 | C2 | 10 May 2015 |
| | | | | AU | 2008308485 | A1 | 09 April 2009 |
| | | | | AU | 2008308485 | B2 | 07 August 2014 |
| | | | | PL | 2214491 | T3 | 30 November 2016 |
| | | | | PT | 3092901 | T | 21 May 2020 |
| | | | | IL | 249906 | A0 | 30 March 2017 |
| | | | | IL | 249906 | B | 29 October 2020 |
| | | | | CA | 2701630 | A1 | 09 April 2009 |
| | | | | CA | 2701630 | C | 25 October 2016 |
| | | | | US | 2009093455 | A1 | 09 April 2009 |
| | | | | US | 7928100 | B2 | 19 April 2011 |
| | | | | US | 2011218184 | A1 | 08 September 2011 |
| | | | | US | 8853234 | B2 | 07 October 2014 |
| | | | | NO | 20100596 | L | 10 June 2010 |
| | | | | BRPI | 0817806 | A2 | 07 October 2014 |
| | | | | PT | 2214491 | T | 25 October 2016 |
| | | | | PL | 3092901 | T3 | 19 October 2020 |
| | | | | DK | 3092901 | T3 | 18 May 2020 |
| | | | | WO | 2009046383 | A1 | 09 April 2009 |
| | | | | NZ | 584892 | A | 29 June 2012 |
| | | | | MX | 2010003685 | A | 02 June 2010 |
| | | | | KR | 20160020578 | A | 23 February 2016 |
| | | | | KR | 101682867 | B1 | 05 December 2016 |
| | | | | EP | 3092901 | A1 | 16 November 2016 |
| | | | | EP | 3092901 | B1 | 29 April 2020 |
| | | | | JP | 2014159474 | A | 04 September 2014 |
| | | | | JP | 2010540663 | A | 24 December 2010 |
| | | | | JP | 5824213 | B2 | 25 November 2015 |
| | | | | IL | 204844 | A0 | 30 November 2010 |
| | | | | IL | 204844 | A | 31 January 2017 |
| | | | | HK | 1150728 | A1 | 13 January 2012 |
| | | | | ES | 2791305 | T3 | 03 November 2020 |
| | | | | KR | 20100064392 | A | 14 June 2010 |
| | | | | KR | 101601332 | B1 | 08 March 2016 |
| | | | | ES | 2582662 | T3 | 14 September 2016 |
| | | | | EP | 2214491 | A1 | 11 August 2010 |
| | | | | EP | 2214491 | A4 | 10 November 2010 |
| | | | | EP | 2214491 | B1 | 13 July 2016 |
| CN | 115919873 | A | 07 April 2023 | | None | | |
| CN | 108601789 | A | 28 September 2018 | JP | 2022020003 | A | 27 January 2022 |
| | | | | AU | 2021225157 | A1 | 30 September 2021 |
| | | | | AU | 2021225157 | B2 | 05 October 2023 |
| | | | | AU | 2016355268 | A1 | 14 June 2018 |
| | | | | AU | 2016355268 | B2 | 19 August 2021 |
| | | | | JP | 2018534321 | A | 22 November 2018 |
| | | | | JP | 7017509 | B2 | 08 February 2022 |
| | | | | AU | 2023229524 | A1 | 28 September 2023 |
| | | | | WO | 2017087235 | A1 | 26 May 2017 |
| | | | | US | 2021113584 | A1 | 22 April 2021 |

Form PCT/ISA/210 (patent family annex) (July 2022)

**INTERNATIONAL SEARCH REPORT**
**Information on patent family members**

International application No.
**PCT/CN2024/089126**

| Patent document cited in search report | Publication date (day/month/year) | Patent family member(s) | Publication date (day/month/year) |
|---|---|---|---|
| | | IL 259423 A | 31 July 2018 |
| | | US 2022088029 A1 | 24 March 2022 |
| | | KR 20180113976 A | 17 October 2018 |
| | | US 2019224209 A1 | 25 July 2019 |
| | | US 11229654 B2 | 25 January 2022 |
| | | TW 201720833 A | 16 June 2017 |
| | | TWI 730013 B | 11 June 2021 |
| | | US 2017143737 A1 | 25 May 2017 |
| | | US 10857156 B2 | 08 December 2020 |
| | | CA 3005730 A1 | 26 May 2017 |
| | | EP 3377068 A1 | 26 September 2018 |
| | | EP 3377068 A4 | 19 June 2019 |
| | | RU 2021121695 A | 23 September 2021 |
| | | RU 2018122172 A | 20 December 2019 |
| | | RU 2018122172 A3 | 12 February 2020 |
| | | RU 2752506 C2 | 28 July 2021 |
| WO 2008131134 A1 | 30 October 2008 | US 2011065687 A1 | 17 March 2011 |
| CN 103533934 A | 22 January 2014 | US 2014086839 A1 | 27 March 2014 |
| | | EP 2685976 A1 | 22 January 2014 |
| | | EP 2685976 B1 | 27 December 2017 |
| | | WO 2012123938 A1 | 20 September 2012 |
| CN 114409681 A | 29 April 2022 | None | |
| WO 2015079411 A1 | 04 June 2015 | CA 2925698 A1 | 04 June 2015 |
| | | CA 2925698 C | 09 November 2021 |
| | | ES 2754207 T3 | 16 April 2020 |
| | | EP 3074391 A1 | 05 October 2016 |
| | | EP 3074391 A4 | 19 April 2017 |
| | | EP 3074391 B1 | 31 July 2019 |
| | | IL 245839 A0 | 02 August 2016 |
| | | IL 245839 B | 31 October 2019 |
| | | US 2017290842 A1 | 12 October 2017 |
| | | US 10022382 B2 | 17 July 2018 |
| | | US 2018264002 A1 | 20 September 2018 |
| | | US 10993948 B2 | 04 May 2021 |
| | | US 2015284410 A1 | 08 October 2015 |
| | | US 9688697 B2 | 27 June 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310438327